# EUROPÄISCHE PATENTSCHRIFT

(11) **EP 0 554 661 B1**
(45) Veröffentlichungstag und Bekanntmachung des Hinweises auf die Patenterteilung: **18.06.1997**
(21) Anmeldenummer: 93100130.9
(22) Anmeldetag: 07.01.1993
(51) Int. Cl.: C07D 239/60, C07D 251/30, C07D 409/12, A01N 43/54, A01N 43/66

(54) **Thiocarbonsäurederivate, Verfahren und Zwischenprodukte zu ihrer Herstellung**
Thiocarboxylic acid derivatives, process and intermediates for their preparation
Dérivés d'acides thiocarboxyliques, procédé et intermédiaires pour leur préparation

(30) Priorität: 24.01.1992 DE 4201875
(43) Veröffentlichungstag der Anmeldung: 11.08.1993
(73) Patentinhaber: BASF Aktiengesellschaft, 67063 Ludwigshafen (DE)
(72) Erfinder: Baumann, Ernst, Dr., W-6720 Speyer (DE); Saupe, Thomas, Dr., W-6902 Sandhausen (DE); Rheinheimer, Joachim, Dr., W-6700 Ludwigshafen (DE); Vogelbacher, Uwe Josef, Dr., W-6700 Ludwigshafen (DE); Bratz, Matthias, Dr., W-6720 Speyer (DE); Meyer, Norbert, Dr., W-6802 Ladenburg (DE); Gerber, Matthias, Dr., 6700 Limburgerhof (DE); Westphalen, Karl-Otto, Dr., W-6720 Speyer (DE); Kardorff, Uwe, Dr., W-6800 Mannheim 1 (DE); Landes, Andreas, Dr., W-6703 Limburgerhof (DE); Walter, Helmut, Dr., W-6719 Obrigheim (DE)

(56) Entgegenhaltungen:
- EP-A- 0 347 811
- EP-A- 0 400 741
- EP-A- 0 409 368

## Beschreibung

Die vorliegende Erfindug betrifft Thiocarbonsäurederivate der allgemeinen Formel I, 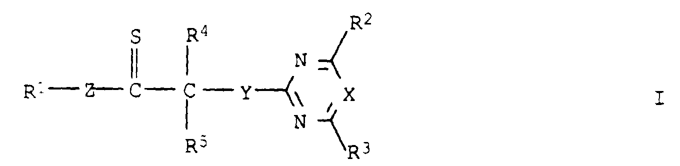 in der die Substituenten folgende Bedeutung haben:
- R¹: eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, C₃-C₁₂-Cycloalkyl, Phenyl, Phenoxy oder Phenylcarbonyl, wobei die Phenylreste ihrerseits ein bis fünf Halogenatome und/oder ein bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
eine C₂-C₆-Alkylgruppe, welche in der 2-Position einen der folgenden Reste trägt: C₁-C₆-Alkoxyimino, C₃-C₆-Alkenyloxyimino, C₃-C₆-Halogenalkenyloxyimino oder Benzyloxyimino;
eine C₃-C₁₂-Cycloalkylgruppe oder C₃-C₁₂-Cycloalkenylgruppe, welche ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
unsubstituiertes oder ein- bis dreifach durch C₁-C₄-Alkyl oder C₁-C₄-Alkoxy substituiertes oder ein- bis fünffach durch Halogen substituiertes Phenyl bedeutet;
- R²: Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR⁶, wobei R⁶ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist und die Alkylen- bzw. Alkenylenkette durch C₁-C₄-Alkyl, Phenyl, C₁-C₄-Alkoxy oder C₁-C₄-Alkoxycarbonyl substituiert sein kann;
- R³: Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R⁶ wie oben angegeben zu einem 5- oder 6-gliedrigen Ring verknüpft;
- R⁴: eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und/oder Phenyl;
eine C₃-C₁₂-Cycloalkyl- oder C₃-C₁₂-Cycloalkenylgruppe, die ein Sauerstoff- oder Schwefelatom enthalten kann und ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche jeweils ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Phenyl oder Naphthyl, welches ein bis fünf Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
R⁴ und R⁵ bilden zusammen mit dem benachbarten Kohlenstoffatom einen 3- bis 6-gliedrigen Ring, der ein Sauerstoff- oder Schwefelatom enthalten kann und einen bis drei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
- R⁵: Wasserstoff, C₁-C₄-Alkyl, C₁-C₄-Halogenalkyl, C₁-C₄-Alkoxy, C₁-C₄-Halogenalkoxy, C₁-C₄-Alkylthio oder R⁵ ist mit R⁴ wie oben angegeben zu einem 3- bis 6-gliedrigen Ring verknüpft;
- Y,Z: Sauerstoff oder Schwefel.

Von der Formel I sind sowohl die racemischen Produkte als auch die optisch aktiven Derivate im Falle ungleicher Reste R⁴ und R⁵ (R-Konfiguration, S-Konfiguration) umfaßt.

In der Literatur (EP-A 347 811, EP-A 400 741, EP-A 409 368) sind herbizid wirksame α-Pyrimidinyloxy(thio)-carbonsäurederivate beschrieben. Die Wirkung und Selektivität dieser Verbindungen ist jedoch nicht immer befriedigend.

Aufgabe der vorliegenden Erfindung war daher die Bereitstellung neuer Carbonsäurederivate mit verbesserten herbiziden und pflanzenwachstumsregulierenden Eigenschaften.

Es wurde nun gefunden, daß die eingangs definierten Thiocarbonsäurederivate der allgemeinen Formel I ausgezeichnete herbizide und pflanzenwachstumsregulierende Eigenschaften besitzen.

Zur Herstellung der erfindungsgemäßen Verbindungen geht man aus von Cyanhydrinen II, die man in allgemein bekannter Weise, z.B. wie in J. March, Advanced Organic Chemistry, 2nd ed., 1983, S. 873 beschrieben, aus den entsprechenden Ketonen bzw. Aldehyden R⁴COR⁵ erhält. Diese Cyanhydrine werden mit etwa äquimolaren Mengen von Pyrimidin- bzw. Triazinderivaten der Formel III, in der W für eine nucleofuge Gruppe wie zum Beispiel Chlor oder R⁷SO₂ wie Methylsulfonyl oder Phenylsulfonyl steht, unter Zuhilfenahme einer organischen oder anorganischen Base in einem inerten organischen Lösungsmittel zu Nitrilen der allgemeinen Formel IV umgesetzt. 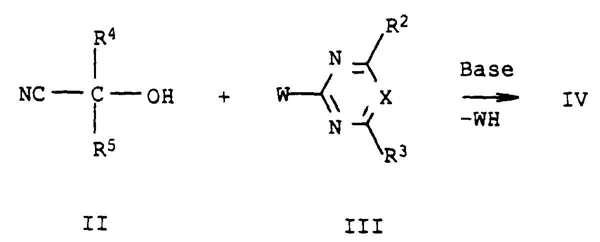 Als Base können Alkali- oder Erdalkalimetallhydride wie NaH und CaH₂, Alkalimetallhydroxide wie NaOH und KOH, Alkalimetallakoholate wie Kalium-tert.-butylat, Alkalimetallcarbonate wie Na₂CO₃ und K₂CO₃, Alkalimetallamide wie NaNH₂ und Lithiumdiisopropylamid oder tertiäre Amine Verwendung finden. Bei Einsatz einer anorganischen Base kann man einen Phasentransferkatalysator zusetzen, wenn dies den Umsatz fördert.

Die Heteroaromaten III sind allgemein bekannt, z.B. aus dem eingangs zitierten Stand der Technik. Verbindungen III, in denen X für eine Gruppe CR⁶ steht und R⁶ zusammen mit R³ einen 5- oder 6-gliedrigen, ggf. einfach ungesättigten, ein Sauerstoffatom enthaltenden Ring bildet, erhält man, indem man ein entsprechendes 2-Alkylthio-5,6-dihydrofuran[2,3]pyrimidin (s. Collect. Czech. Chem. Commun. 32, 1582 (1967)) 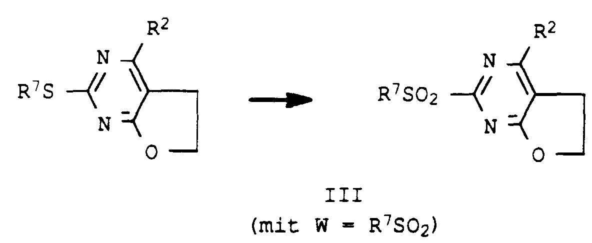 durch Oxidationsmittel wie z.B. Chlor in Wasser oder Wasserstoffperoxid in Eisessig unter milden Bedingungen oxidiert.

R⁷SO₂ in Formel III bedeutet eine übliche nucleofuge Abgangsgruppe, beispielsweise Arylsulfonyl wie Phenyl- oder substituiertes Phenylsulfonyl, wobei als Substituenten ein oder mehrere, z.B. 1 bis 3 niedermolekulare Alkyl- oder Alkxyreste wie C₁-C₄-Alkyl oder Alkoxy oder Halogen, z.B. Chlor, Fluor oder Brom in Betracht kommen; oder Alkylsulfonyl wie C₁-C₄-Alkylsulfonyl, z.B. Methylsulfonyl.

Die Herstellung anellierter Pyrimidine ist weiterhin beispielsweise beschrieben in

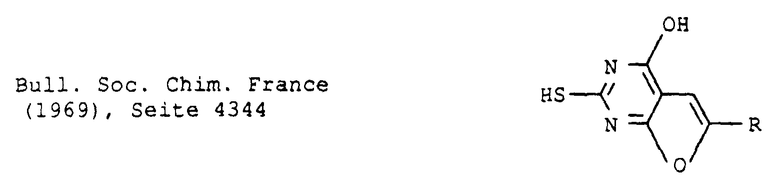

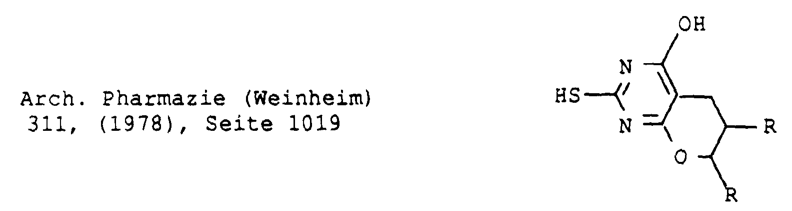

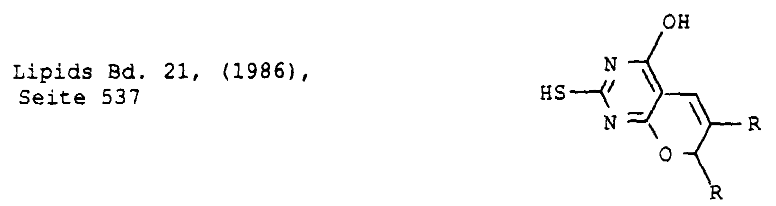

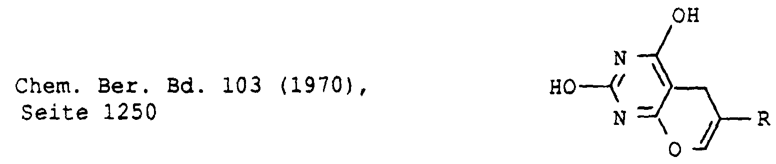

Durch Behandeln mit überschüssigem Schwefelwasserstoff in einem inerten Lösungsmittel erhält man aus den Nitrilen IV die entsprechenden Thioamide V, 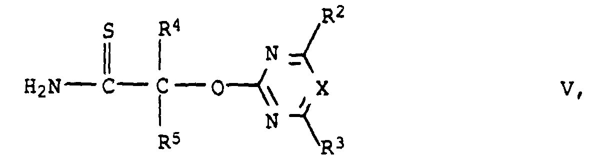 die mit einer Verbindung VI

R¹A VI,

wobei R¹ die oben genannte Bedeutung hat und A eine nukleofuge Abgangsgruppe wie zum Beispiel Halogen, Alkylsulfat oder Dialkyloxoniumtetrafluoroborat darstellt, in einem inerten Lösungsmittel umgesetzt werden und anschließend durch Behandeln mit überschüssigem Schwefelwasserstoff die gewünschten Verbindungen der Formel I mit Y = O liefern.

Üblicherweise wird ein geringer Überschuß an VI, bezogen auf die Thioamide V, z.B. 1,05 bis 1,2 Äquivalente VI, verwendet.

Alternativ dazu lassen sich die erfindungsgemäßen Verbindungen erhalten, indem man die z.B. aus EP-A 347 811, EP-A 400 741 oder EP-A 409 368 bekannten Carbonsäurederivate der Formel VII, 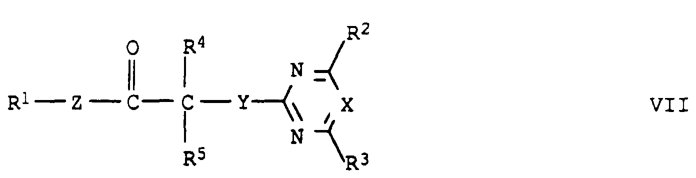 in der die Substituenten die oben genannte Bedeutung haben, mit Lawesson's Reagenz (Bull. Soc. Chim. Belg. 87(1987)293) umsetzt.

Im Hinblick auf die biologische Wirkung sind Thiocarbonsäurederivate I bevorzugt, in denen die Substituenten folgende Bedeutung haben:
- R¹: C₁-C₁₀-Alkyl, insbesondere C₁-C₈-Alkyl wie Methyl, Ethyl, Propyl, 1-Methylethyl, Butyl, 1-Methylpropyl, 2-Methylpropyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Diemthylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl; welches ein bis fünf Halogenatome wie Fluor, Chlor, Brom, Jod, insbesondere Fluor und Chlor, und/oder einen der folgenden Reste tragen kann: Cyano,
C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
C₁-C₈-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimetylpropylcarbonyl, 1,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
C₁-C₈-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl, n-Pentyloxycarbonyl, 1-Methylbutyloxycarbonyl, 2-Methylbutyloxycarbonyl, 3-Methylbutyloxycarbonyl, 1,2-Dimethylpropyloxycarbonyl, 1,1-Dimethylpropyloxycarbonyl, 2,2-Dimethylpropyloxycarbonyl, 1-Ethylpropyloxycarbonyl, n-Hexyloxycarbonyl, 1-Methylpentyloxycarbonyl, 2-Methylpentyloxycarbonyl, 3-Methylpentyloxycarbonyl, 4-Methylpentyloxycarbonyl, 1,2-Dimethylbutyloxycarbonyl, 1,3-Dimethylbutyloxycarbonyl, 2,3-Dimethylbutyloxycarbonyl, 1,1-Dimethylbutyloxycarbonyl, 2,2-Dimethylbutyloxycarbonyl, 3,3-Dimethylbutyloxycarbonyl, 1,1,2-Trimethylpropyloxycarbonyl, 1,2,2-Trimethylpropyloxycarbonyl, 1-Ethylbutyloxycarbonyl, 2-Ethylbutyloxycarbonyl, 1-Ethyl-2-methylpropyloxycarbonyl, n-Heptyloxycarbonyl, 1-Methylhexyloxycarbonyl, 2-Methylhexyloxycarbonyl, 3-Methylhexyloxycarbonyl, 4-Methylhexyloxycarbonyl, 5-Methylhexyloxycarbonyl, 1-Ethylpentyloxycarbonyl, 2-Ethylpentyloxycarbonyl, 1-Propylbutyloxycarbonyl und Octyloxycarbonyl, insbesondere Methoxycarbonyl, Ethoxycarbonyl, 1-Methylethoxycarbonyl und 1-Methylpropyloxycarbonyl;
C₃-C₁₂-Cycloalkyl, insbesondere C₃-C₆-Cycloalkyl wie Cyclopropyl, Cyclobutyl, Cyclopentyl, Cyclohexyl;
Phenyl, Phenoxy, Phenylcarbonyl, wobei die Phenylreste ihrerseits ein bis fünf Halogenatome, wie oben genannt, und/oder ein bis drei der folgenden Reste tragen können:
C₁-C₄-Alkyl wie Methyl, Ethyl, 1-Propyl, 2-Propyl, 2-Methyl-2-propyl, 2-Methyl-1-propyl, 1-Butyl, 2-Butyl;
C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
- R¹: ferner C₁-C₁₀-Alkyl, insbesondere C₁-C₄-Alkyl wie vorstehend genannt, welches ein bis fünf Halogenatome wie vorstehend genannt, vorzugsweise Fluor und Chlor, tragen kann und durch einen der folgende Reste substituiert ist:
5-gliedriges Heteroaryl wie Furyl, Thienyl, Pyrryl, Pyrazolyl, Imidazolyl, Triazolyl, Isoxazolyl, Oxazolyl, Isothiazolyl, Thiazolyl, Thiadiazolyl, welches seinerseits ein bis vier Halogenatome wie vorstehend genannt, vorzugsweise Fluor und Chlor und/oder einen bis zwei der folgenden Reste tragen kann: C₁-C₄-Alkyl, C₁-C₄-Alkoxy, C₁-C₄-Alkoxy, C₁-C₄-Halogenalkyl, C₁-C₄-Halogenalkoxy, C₁-C₄-Alkylthio wie im einzelnen oben genannt;
- R¹: ferner C₂-C₆-Alkyl, insbesondere C₂-C₄-Alkyl, das in 2-Stellung substituiert ist durch C₁-C₆-Alkoxyimino, z.B. Methoxy-, Ethoxy- oder Propoxyimino; C₃-C₆-Alkenyloxyimino wie 2-Propenyloxyimino, 2-Butenyloxyimino, 3-Butenyloxyimino; C₃-C₆-Halogenalkenyloxyimino wie 3,3-Dichlor-2-propenyloxyimino, 2,3,3-Trichlor-2-propenoxyimino oder Benzyloxyimino;
- R¹: ferner C₃-C₁₂-Cycloalkyl, insbesondere C₃-C₇-Cycloalkyl wie Cyclopropyl, Cyclobutyl, Cyclopentyl, Cyclohexyl, Cycloheptyl, welches ein bis fünf Halogenatome wie vorstehend genannt, insbesondere Fluor und Chlor und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im einzelnen voranstehend genannte, wobei die Phenylreste ihrerseits wie angegebenen substituiert sein können durch Halogen, insbesondere Fluor oder Chlor, Alkyl, Halogenalkyl, Alkoxy, Halogenalkoxy und/oder Alkylthio;
C₃-C₁₂-Cycloalkenyl, insbesondere C₄-C₇-Cycloalkenyl wie Cyclobutenyl, Cyclopentenyl, Cyclohexenyl, Cycloheptenyl, welches wie die Cycloalkylgruppe substituiert sein kann durch Halogen, Alkyl, Alkoxy, Alkylthio, Cyano, Alkylcarbonyl, Alkoxycarbonyl, Phenyl, Phenoxy, Phenylcarbonyl;
C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-2-butenyl, 2,2-Dimethyl-3-butenyl, 2,2-Dimethyl-3-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
C₃-C₆-Alkinyl wie 2-Propinyl, 2-Butinyl, 3-Butinyl, 1-Methyl-2-propinyl, 2-Pentinyl, 3-Pentinyl 4-Pentinyl, 1-Methyl-3-butinyl, 2-Methyl-3-butinyl, 1-Methyl-2-butinyl, 1,1-Dimethyl-2-propinyl, 1-Ethyl2-propinyl, 2-Hexinyl, 3-Hexinyl, 4-Alkinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl und 1-Methyl-2-butinyl, insbesondere 2-Propinyl, wobei diese Alkenyl- und Alkinylgruppen ein bis fünf Halogenatome tragen können und/oder einen der folgenden Reste: Alkoxy, Alkylthio, Cyano, Alkylcarbonyl, Alkoxycarbonyl, Phenyl, Phenoxy, Phenylcarbonyl wie vorstehend genannt;
- R¹: ferner Phenyl oder Phenyl ein- bis dreifach substituiert durch C₁-C₄-Alkyl oder C₁-C₄-Alkoxy wie Methyl, Ethyl, Propyl, Butyl, Methoxy, Ethoxy, Propoxy, Butoxy oder Phenyl substituiert durch ein bis fünf Halogenatome, z.B. Chlor oder Fluor;
- R²: die bei R¹ im einzelnen genannten Alkyl-, Halogenalkyl-, Alkoxy-, Halogenalkoxy-, Alkylthiogruppen und Halogenatome bedeutet, insbesondere Chlor, Methyl, Methoxy, Ethoxy, Difluormethoxy, Trifluormethoxy;
- X: Stickstoff oder CR⁶, worin
- R⁶: Wasserstoff bedeutet oder zusammen mit R³ eine 3- bis 4-gliedrige Alkylen- oder Alkenylenkette bildet, in der jeweils eine Methylengruppe durch Sauerstoff ersetzt ist wie -CH₂-CH₂-O-, -CH=CH-O-, -CH₂-CH₂-CH₂-O-, -CH=CH-CH₂-O-, insbesondere Wasserstoff und -CH₂-CH₂-O-; diese durch Sauerstoff unterbrochene Alkylen- bzw. Alkenylenkette kann zusätzlich ein- oder mehrere, z.B. ein oder zwei Substituenten, ausgewählt aus der Gruppe C₁-C₄-Alkyl, Phenyl, C₁-C₄-Alkoxy, C₁-C₄-Alkoxycarbonyl, z.B. Methyl, Methoxy, Ethyl, Ethoxy, Methoxycarbonyl oder Ethoxycarbonyl, enthalten;
- R³: die bei R¹ genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome bedeutet, insbesondere Chlor, Methyl, Methoxy, Ethoxy, Difluormethoxy, Trifluormethoxy; oder mit R⁶ wie oben genannt zu einem 5- oder 6-gliedrigen Ring verknüpft ist;
- R⁴: C₁-C₁₀-Alkyl wie bei R¹ im einzelnen genannt, welches ein bis fünf Halogenatome wie Fluor, Chlor, Brom, Jod, insbesondere Fluor und Chlor und/oder einen der folgenden Reste tragen kann: Alkoxy, Alkylthio, Cyano, Alkylcarbonyl, Alkoxycarbonyl, Phenyl, Phenoxy, Phenylcarbonyl wie im allgemeinen und besonderen bei R¹ genannt;
C₁-C₁₀-Alkyl wie vorstehend genannt, welches ein bis fünf Halogenatome wie vorstehend genannt, insbesondere Fluor und Chlor, tragen kann und einen ggf. substituierten 5-gliedrigen Heteroaromaten, wie voranstehend für R¹ genannt, trägt;
C₃-C₁₂-Cycloalkyl, insbesondere C₃-C₇-Cycloalkyl oder C₃-C₁₂-Cycloalkenyl, insbesondere C₄-C₇-Cycloalkenyl, wobei im gesättigten oder ungesättigten Ring eine Methylengruppe durch ein Sauerstoff- oder Schwefelatom ersetzt sein kann, wie Cyclopropyl, Cyclobutyl, Cyclopentyl, Cyclohexyl, Cycloheptyl, Tetrahydrofuranyl, Tetrahydrothienyl, Tetrahydropyranyl, Tetrahydrothiopyranyl; Cyclopropenyl, Cyclobutenyl, Cyclopentenyl, Cyclohexenyl, Cycloheptenyl, Dihydrofuranyl, Dihydrothienyl, Dihydropyranyl, Dihydrothiopyranyl, wobei die Cycloalkyl- bzw. Cycloalkenylreste substituiert sein können durch ein bis fünf Halogenatome wie vorstehend genannt, insbesondere Fluor oder Chlor und/oder einen der folgenden Reste: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im allgemeinen und besonderen oben genannt;
C₃-C₆-Alkenyl oder C₃-C₆-Alkinyl wie bei R¹ genannt, welche ein bis fünf Halogenatome wie vorstehend genannt, insbesondere Fluor und Chlor und/oder einen der folgenden Reste tragen können:
C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im allgemeinen und besonderen oben genannt;
5- oder 6-gliedriges Heteroaryl wie Furyl, Thienyl, Pyrryl, Pyrazolyl, Imidazolyl, Triazolyl, Isoxazolyl, Oxazolyl, Isothiazolyl, Thiazolyl, Thiadiazolyl, Pyridyl, Pyrimidinyl, Pyrazinyl, Pyridazinyl, Triazinyl, beispielsweise 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazoly, 2-Thiazolyl, 4-Thiazolyl, 5-Thiazolyl, 2-Imidazolyl, 4-Imidazolyl, 5-Imidazolyl, 2-Pyrrolyl, 3-Pyrrolyl, 3-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazolyl, Thia-2,4-diazolyl, Thia-3,4-diazolyl und Triazolyl; wobei die Heteroaromaten ein bis fünf Halogenatome wie vorstehend genannt, insbesondere Fluor und Chlor und/oder einen der folgenden Reste tragen können:
C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy, Phenylcarbonyl wie im allgemeinen und besonderen oben genannt;
Phenyl oder Naphthyl, welches ein bis fünf Halogenatome wie vorstehend genannt, insbesondere Fluor und Chlor und/oder einen bis zwei der folgenden Reste tragen kann:
C₁-C₄-Alkyl, C₁-C₄-Alkoxy, C₁-C₄-Halogenalkyl, C₁-C₄-Halogenalkoxy, C₁-C₄-Alkylthio wie im allgemeinen und besonderen oben genannt;
oder R⁴ bildet mit R⁵ zusammen mit dem benachbarten Kohlenstoffatom einen 3-bis 6-gliedrigen Ring, der ein Sauerstoff- oder Schwefelatom enthalten kann und unsubstituiert ist oder je nach Ringgröße einen bis drei der folgenden Reste trägt:
C₁-C₄-Alkyl, C₁-C₄-Alkoxy, C₁-C₄-Halogenalkyl, C₁-C₄-Halogenalkoxy, C₁-C₄-Alkylthio wie im allgemeinen und besonderen oben genannt;
- R⁵: C₁-C₄-Alkyl, C₁-C₄-Alkoxy, C₁-C₄-Halogenalkyl, C₁-C₄-Halogenalkoxy, C₁-C₄-Alkylthio wie im allgemeinen und besonderen oben für R¹ genannt, ferner Wasserstoff, oder R⁴ bildet mit R⁵ einen 3- bis 6-gliedrigen Ring wie oben angegeben;
- Y: Sauerstoff oder Schwefel,
- Z: Sauerstoff oder Schwefel.

Insbesondere bevorzugt sind Verbindungen der Formel I, in der R¹ C₁-C₄-Alkyl, z.B. Methyl oder Ethyl, R² C₁-C₄-Alkoxy, z.B. Methoxy, R⁵ Wasserstoff, Y Sauerstoff bedeuten, und X, Z, R³ und R⁴ die oben genannte Bedeutung haben.

R³ steht besonders bevorzugt für C₁-C₄-Alkoxy, z.B. Methoxy oder Ethoxy, Halogen, z.B. Chlor oder C₁-C₄-Alkyl, z.B. Methyl oder Ethyl oder bildet im Fall von X = CR⁶ mit R⁶ zusammen eine -OCH₂CH₂-Kette.

X steht bevorzugt für N, CH oder CR⁶, wobei R⁶ mit R³ wie bei R³ angegeben verknüpft ist.

Beispiele für besonders bevorzugte Verbindungen der allgemeinen Formel I sind in den folgenden Tabellen a und b zusammengestellt:

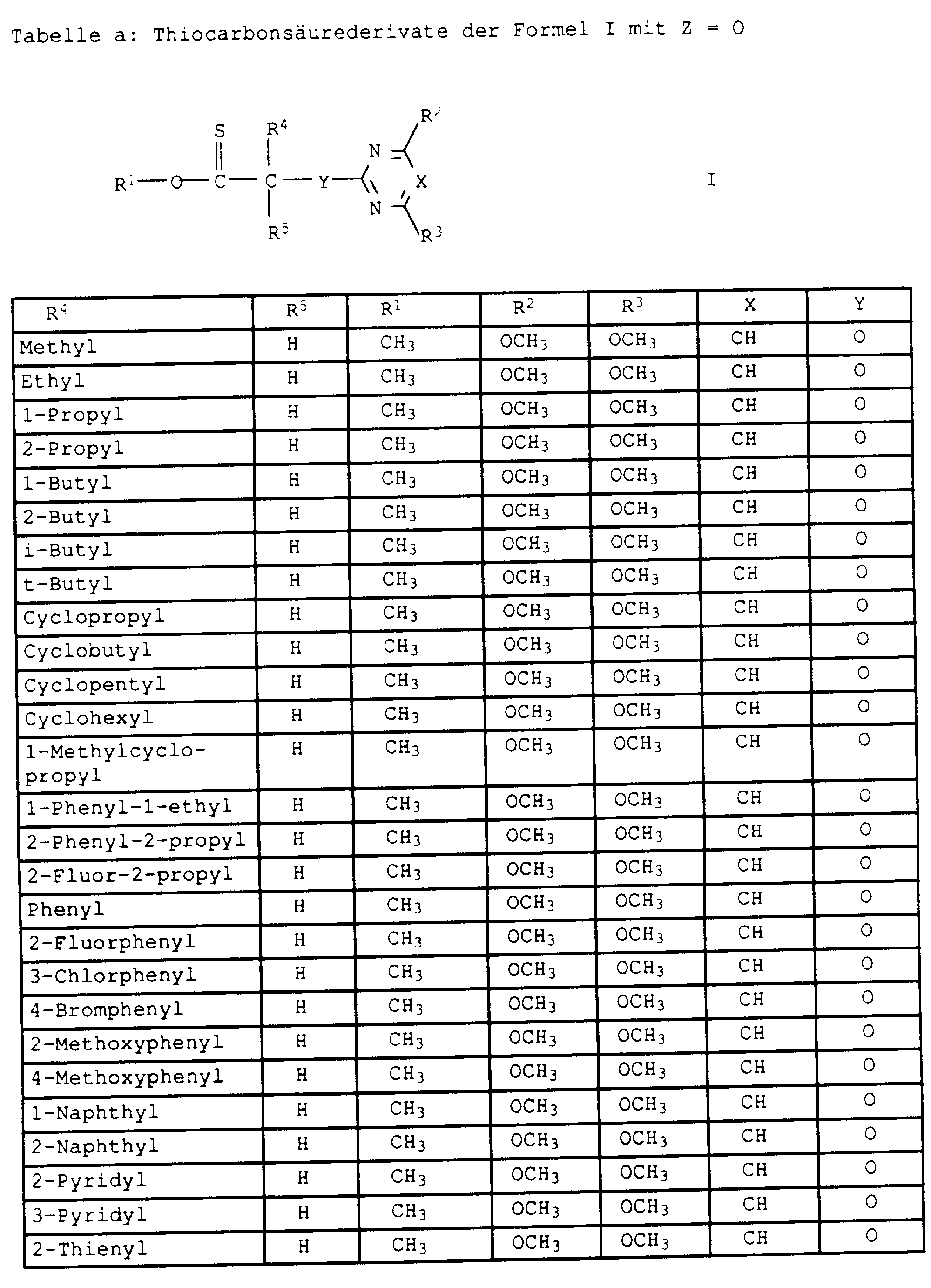

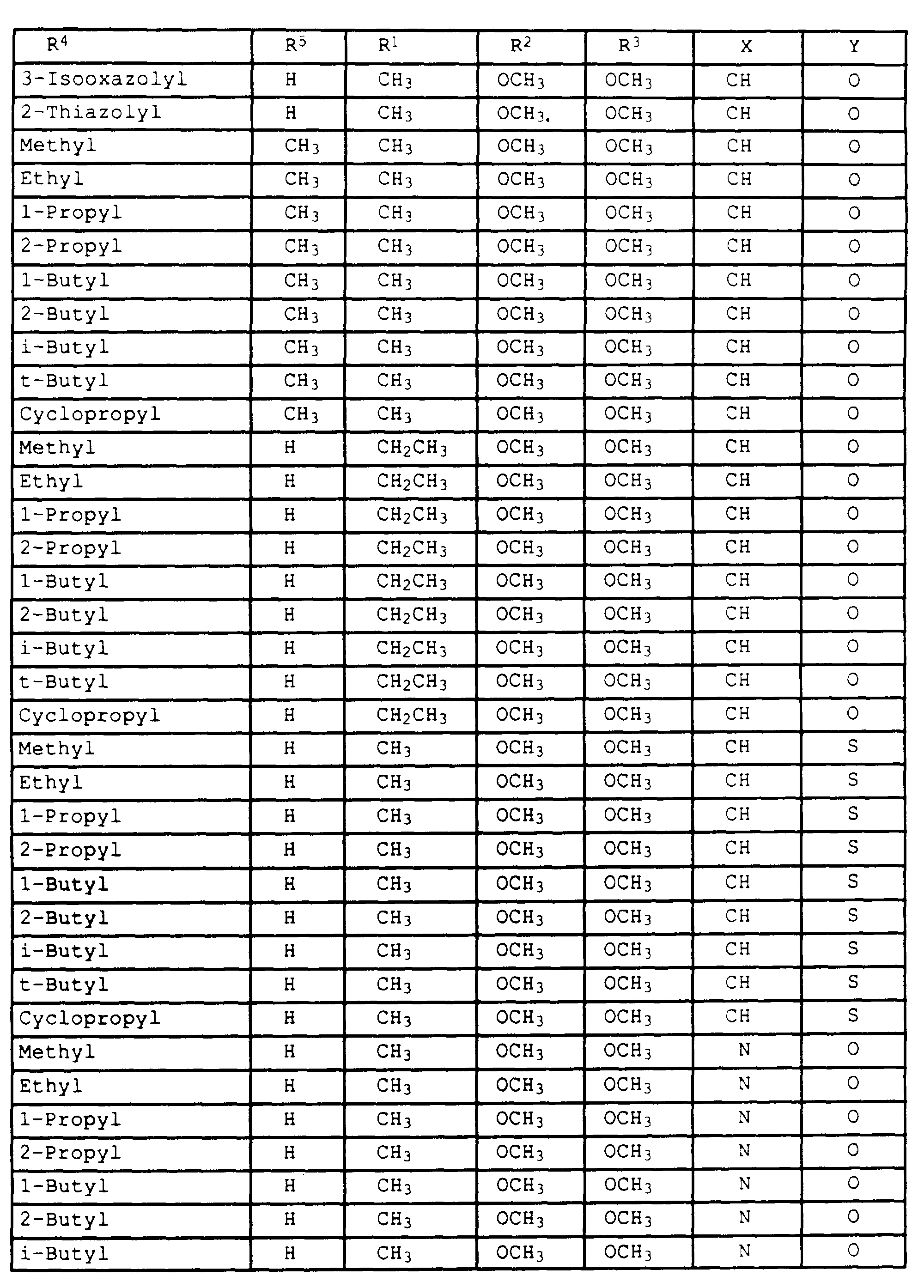

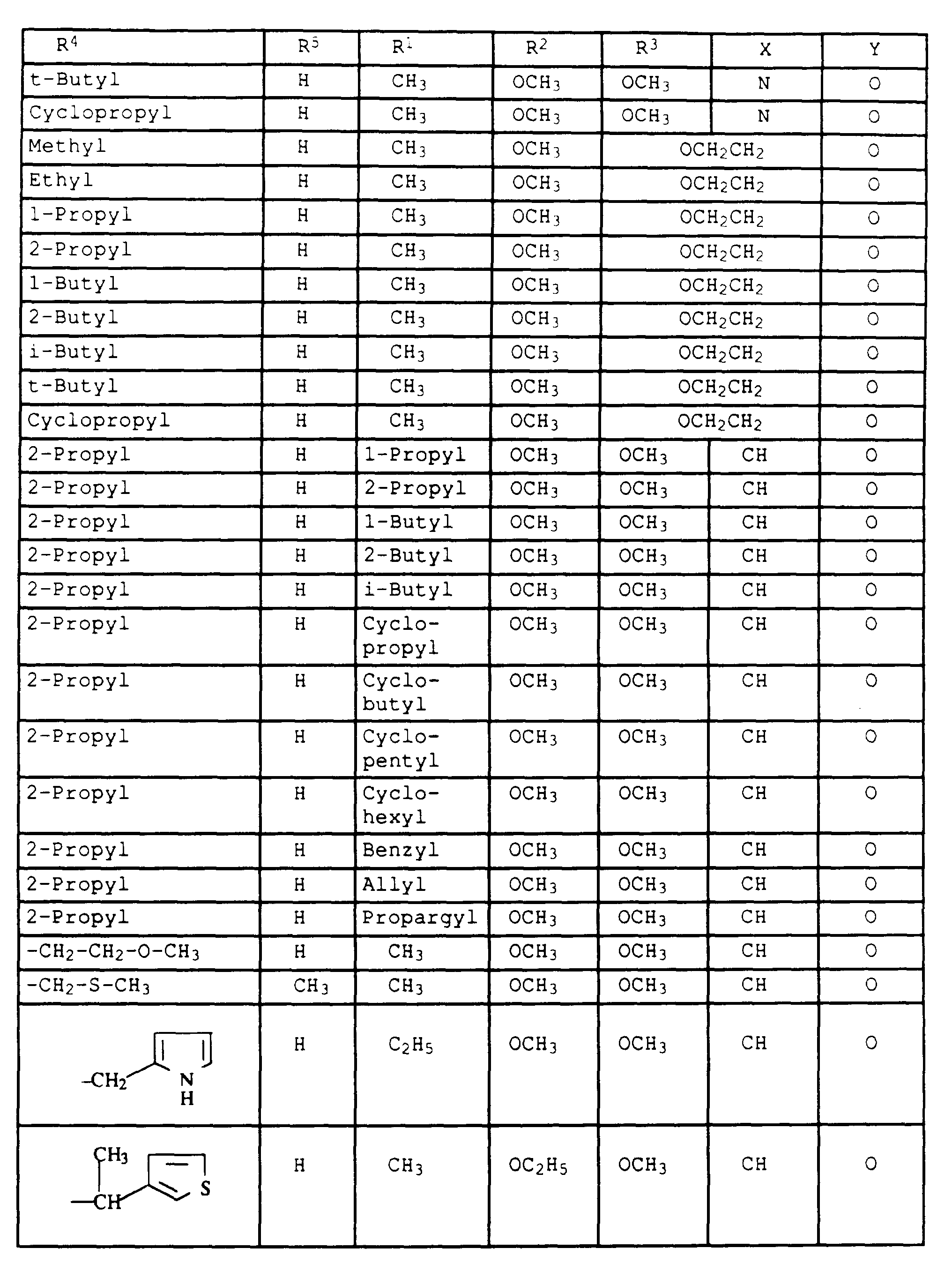

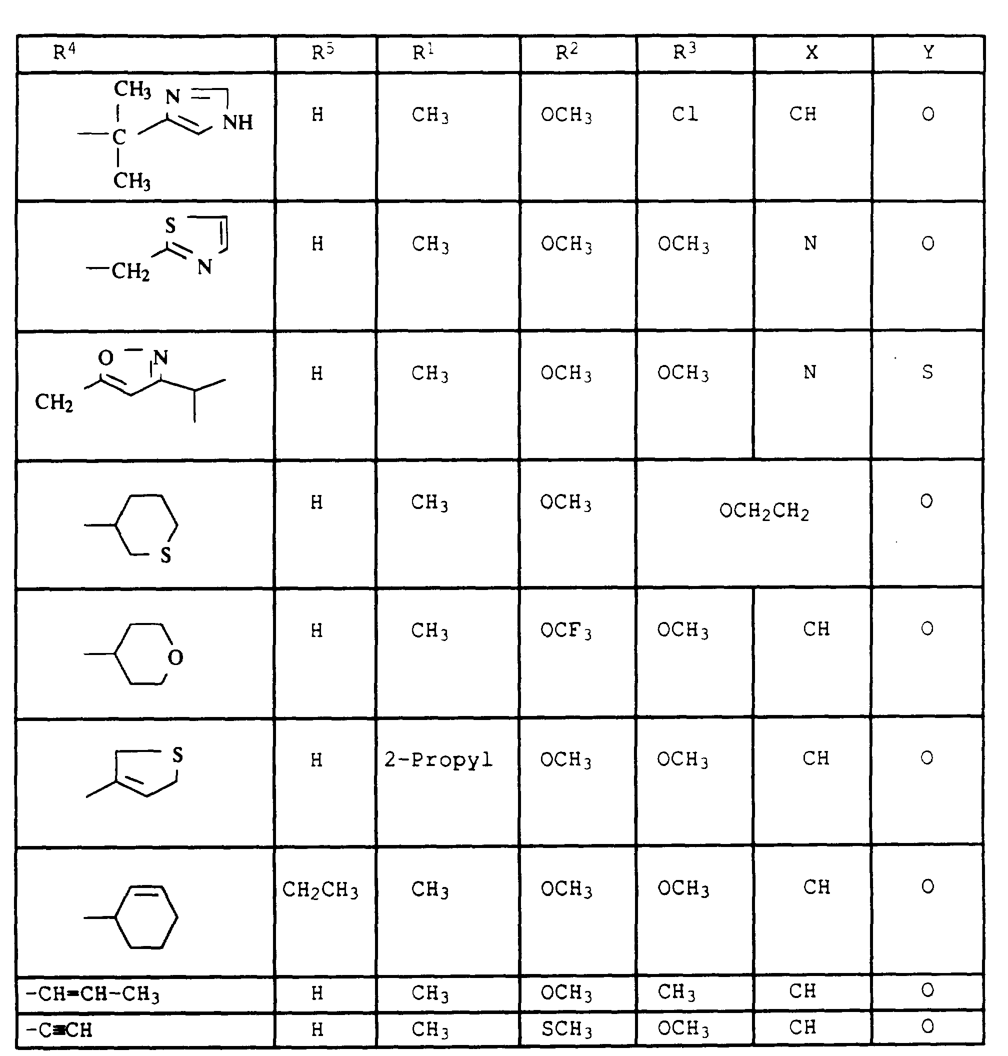

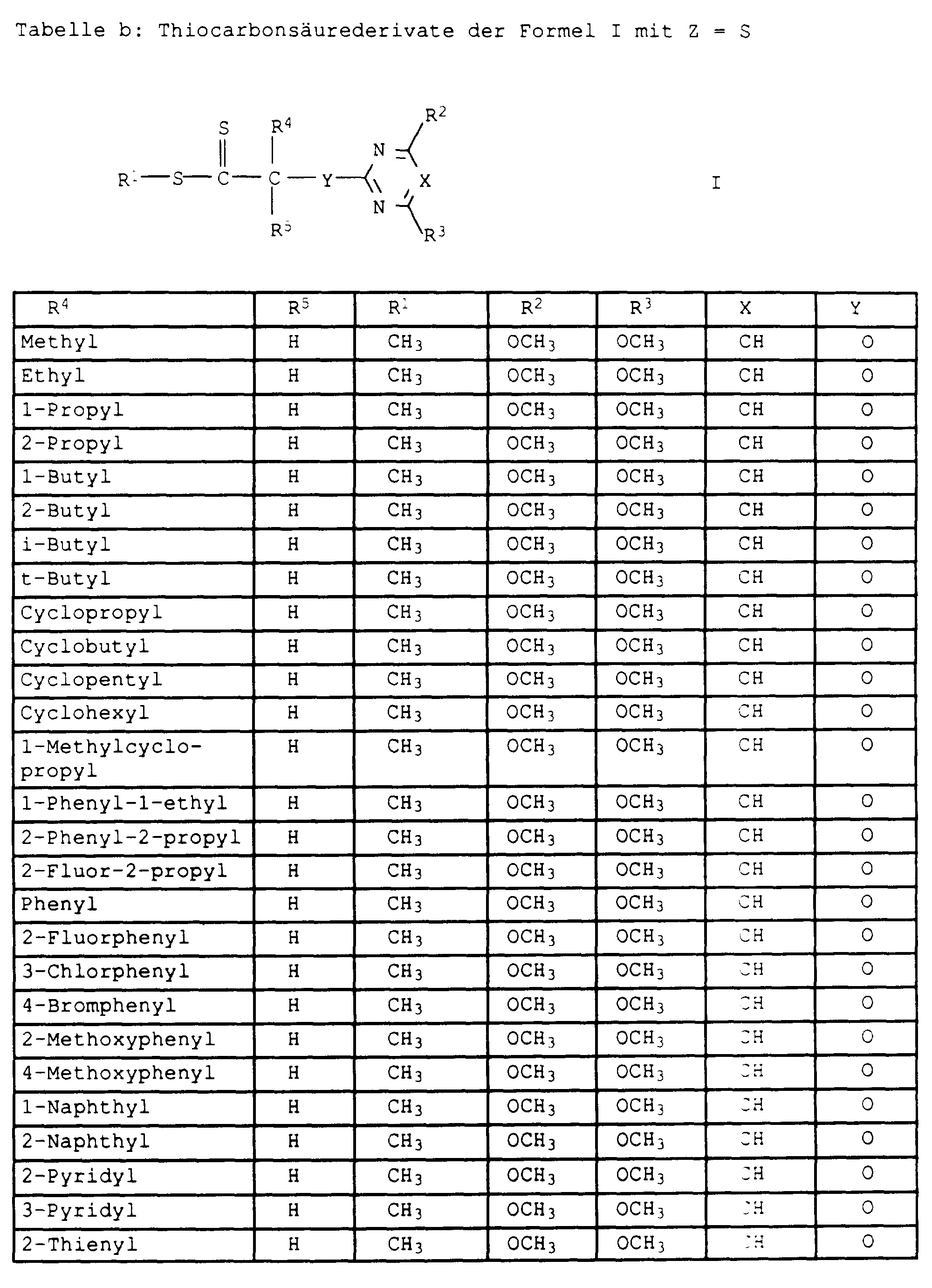

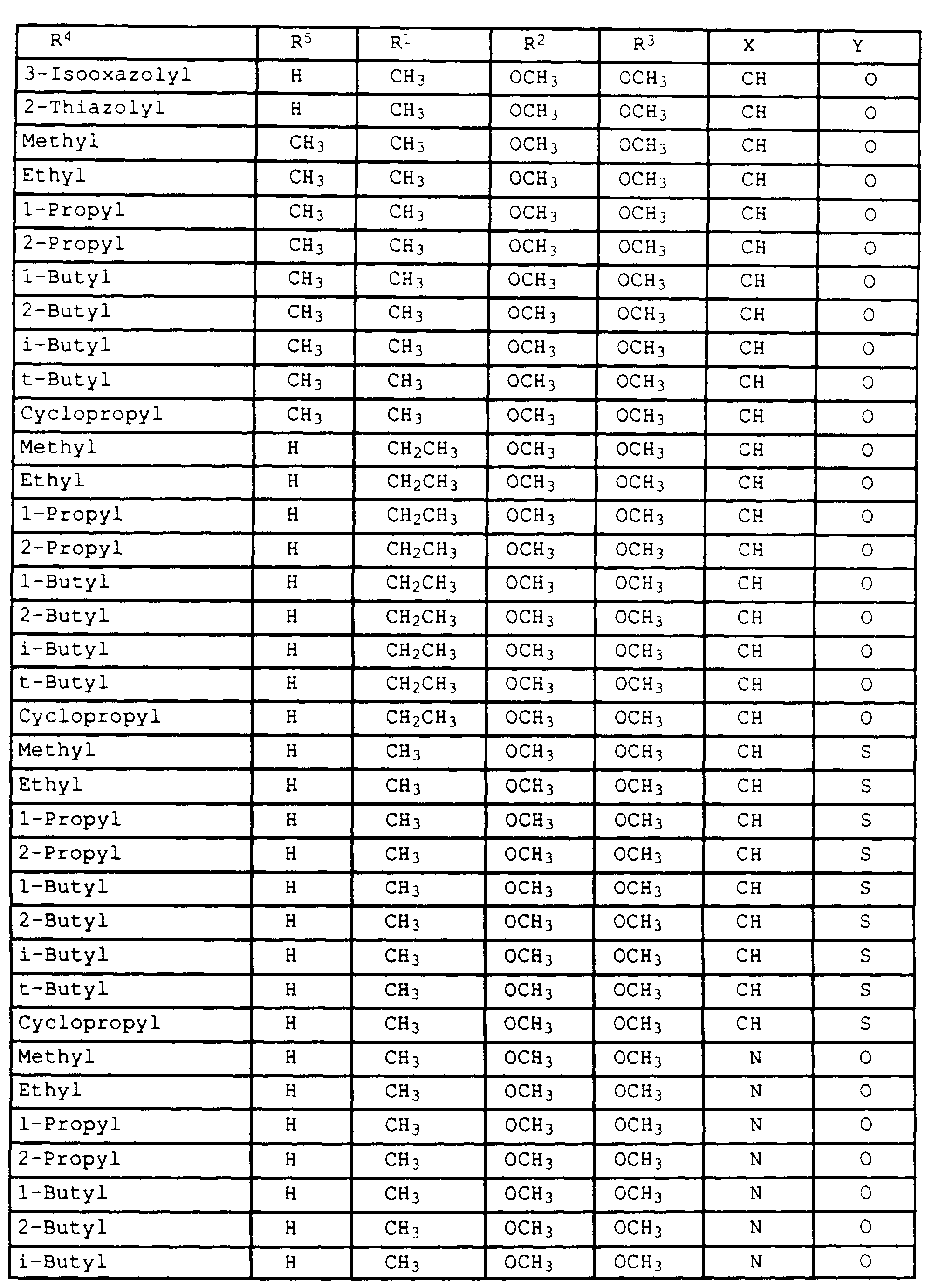

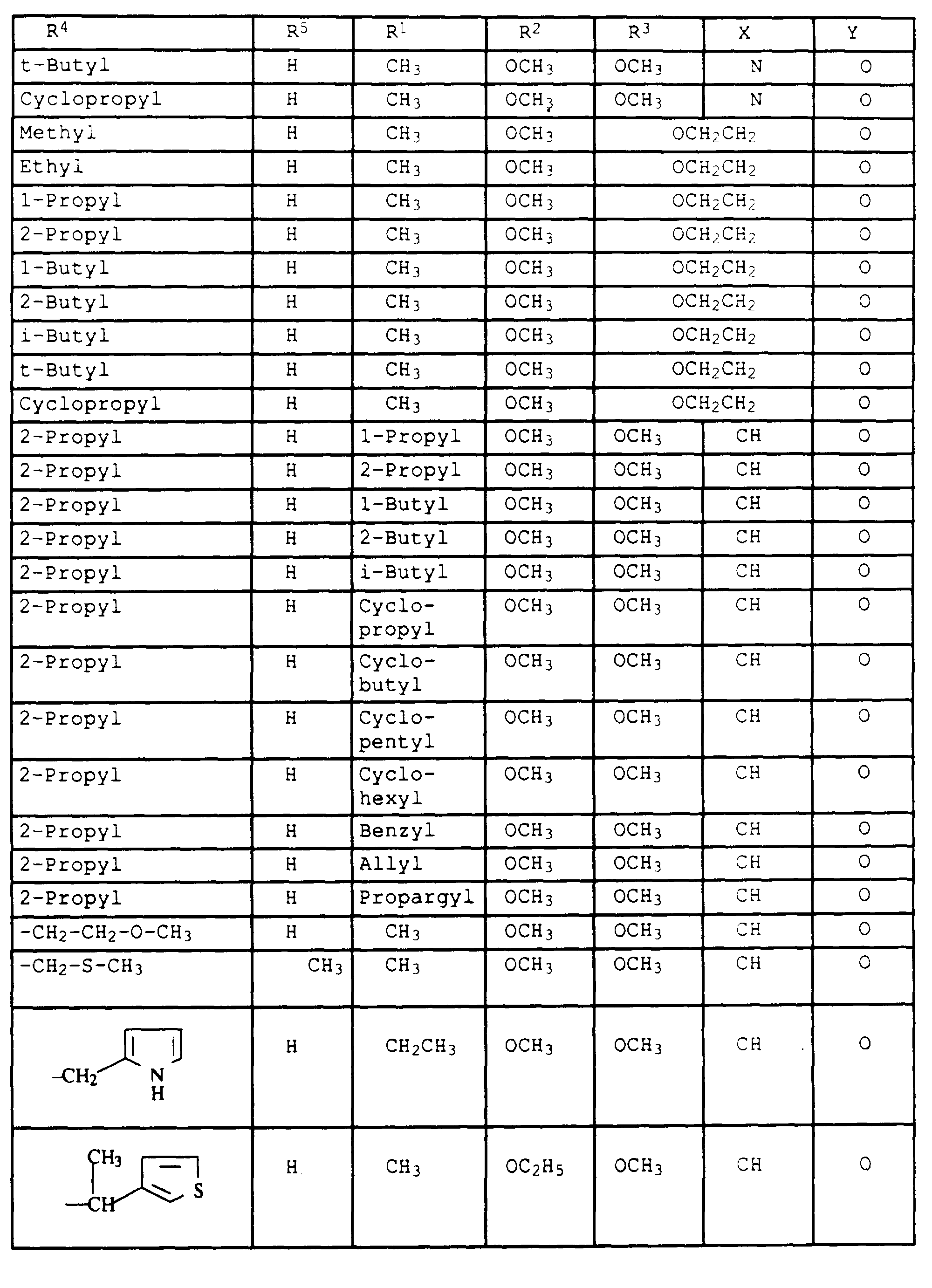

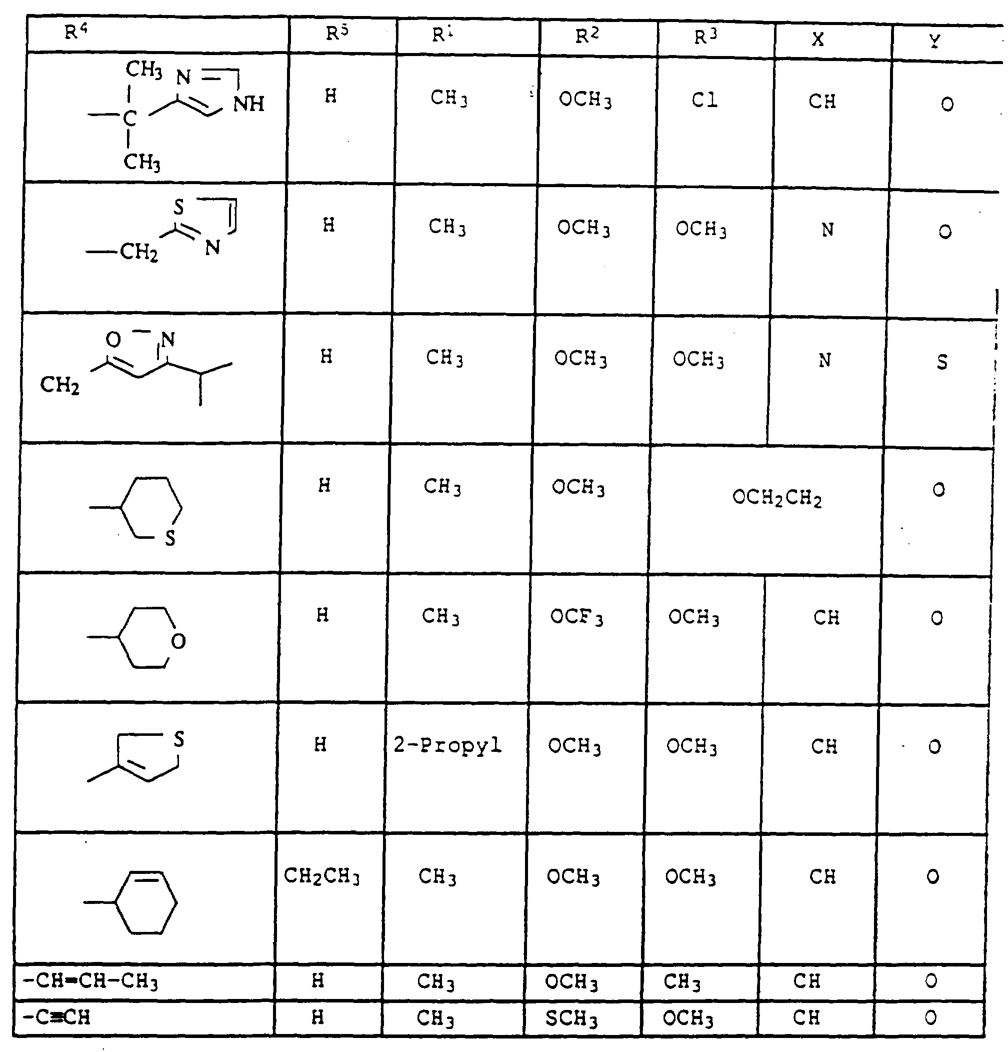

Die Verbindungen der allgemeinen Formel I bzw. die sie enthaltenden herbiziden oder wachstumsregulierenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, vernetzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetal, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,01 und 95 Gew.-%, vorzugsweise zwischen 0,5 und 90 Gew.-%, Wirkstoff. Die Wirkstoffe werden dabei in einer Reinheit von 90 bis 100 %, vorzugsweise 95 bis 100 % (nach NMR-Spektrum) eingesetzt.

Die erfindungsgemäßen Verbindungen I können beispielsweise wie folgt formuliert werden:
I. Man vermischt 90 Gew.-Teilen der Verbindung Nr. 1.001 mit 10 Gew.-Teilen N-Methyl-α-pyrrolidon und erhält eine Lösung, die zur Anwendung in Form kleinster Tropfen geeignet ist.
II. 20 Gew.-Teile der Verbindung Nr. 1.002 werden in einer Mischung gelöst, die aus 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl besteht. Durch Ausgießen und feines Verteilen der Lösung in 100.000 Gew.-Teilen Wasser erhält man eine wäßrige Dispersion, die 0,02 Gew.-% des Wirkstoffs enthält.
III. 20 Gew.-Teile der Verbindung Nr. 1.007 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
IV. 20 Gew.-Teile des Wirkstoffs Nr. 1.008 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.
V. 20 Gew.-Teile des Wirkstoffs Nr. 1.009 werden mit 3 Gew.-Teilen des Natriumsalzes der Diisobutylnaphtalin-α-sulfonsäure, 1 Gew.-Teil des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.
VI. 3 Gew.-Teile des Wirkstoffs Nr. 1.004 werden mit 97 Gew.-Teilen feinteiligem Kaolin vermischt. Man erhält auf diese Weise ein Stäubemittel, das 3 Gew.-% des Wirkstoffs enthält.
VII. 30 Gew.-Teile des Wirkstoffs Nr. 1.003 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Gew.-Teile des Wirkstoffs Nr. 1.001 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenolsulfonsäureharnstoff-formaldehyd-Kondensates und 68 Gew.-Teilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich oder sollen diese Pflanzen möglichst unbeeinflußt weiterwachsen, so können Ausbringungstechniken angewandt werden, bei welchen die Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3, vorzugsweise 0,01 bis 2 kg/ha aktive Substanz (a.S.).

Die Verbindungen der Formel I können praktisch alle Entwicklungsstadien einer Pflanze verschiedenartig beeinflussen und werden deshalb auch als Wachstumsregulatoren eingesetzt. Die Wirkungsvielfalt der Pflanzenwachstumsregulatoren hängt aber vor allem
a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.
   Als vorteilhaft für die Praxis erweist sich eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.
   Von wirtschaftlichem Interesse ist auch die Erhöhung der Standfestigkeit von lageranfälligen Kulturen wie Getreide, Reis, Mais, Sonnenblumen und Soja. Die dabei verursachte Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stomata verändert wird
   - eine dickere Epidermis und Cuticula ausgebildet werden
   - die Durchwurzelung des Bodens verbessert wird und
   - das Mikroklima im Pflanzenbestand durch einen kompakteren Wuchs günstig beeinflußt wird.
E. Mit den Wachstumsregulatoren der Formel I kann ferner das Wachstum von unerwünschten Pflanzen eingeschränkt werden. In Ackerkulturen kann so die Konkurrenzfähigkeit von Unkräutern und Schadgräsern gegenüber den Kulturpflanzen reduziert werden, ohne daß dabei eine direkte herbizide Wirkung vorliegt. Ebenso ist es z.B. in Obst- oder Rebenanlagen möglich, die Konkurrenzkraft des Unterwuchses zum Vorteil der Nutzpflanzen stark zu vermindern ohne dabei die entsprechenden Gräser und Kräuter abzutöten. Auf diese Weise wird eine geschlossene Bodenbedeckung und damit z.B. eine bessere Bodenbelebtheit und ein Schutz vor Erosion erzielt.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen und zur Regulierung des Pflanzenwuchses eingesetzt werden. In Betracht kommen beispielsweise folgende Kulturen:

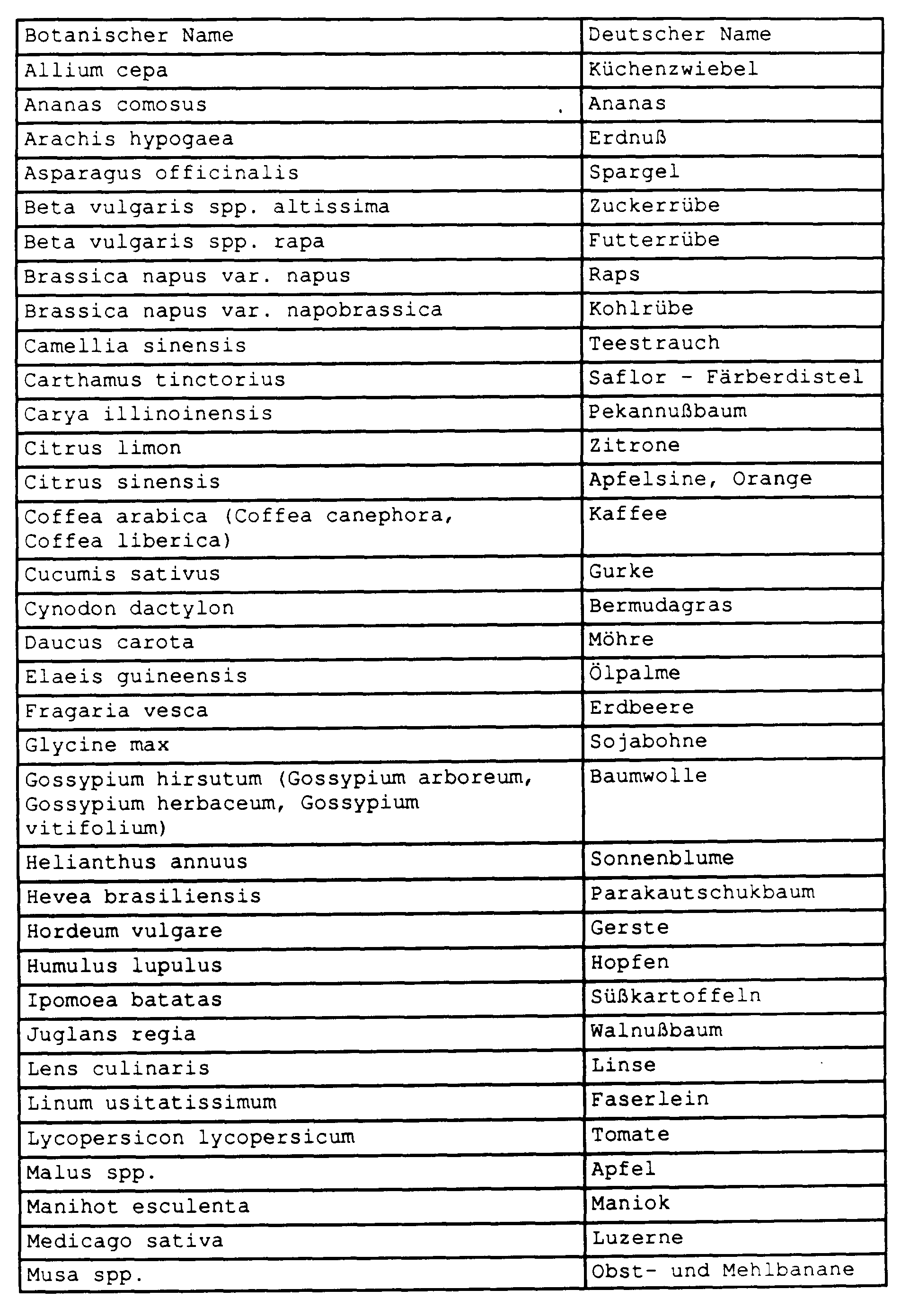

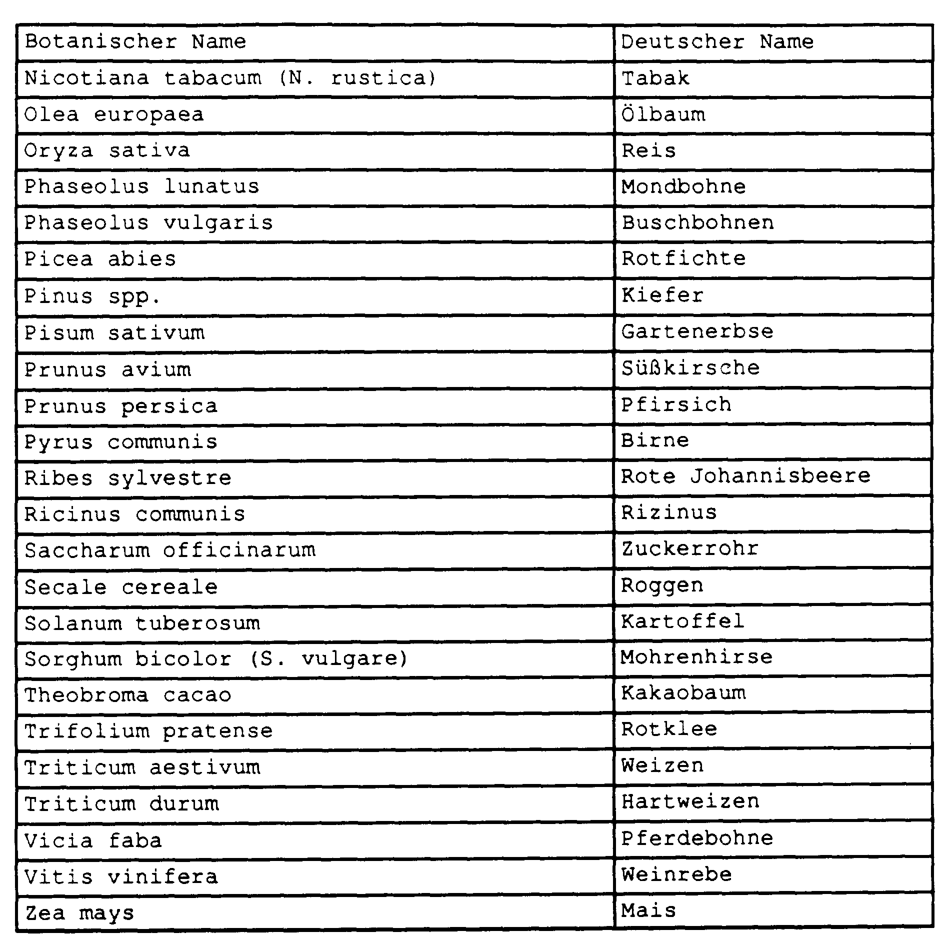

Zur Verbreiterung des Wirkungsspektrums und zur Erzielung synergistischer Effekte können die Thiocarbonsäurederivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Als weitere Wachstumsregulatoren kommen neben anderen in Betracht: Quaternäre Ammoniumverbindungen, Triazole, Cyclohexantrione, Imidazole, Norbornanodiazetine, 4-Pyridine, Ethephon, Abscisinsäure und davon abgeleitete Strukturen.

Außerdem kann es von Nutzen sein, die neuen Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nicht-phytotoxische Öle und Ölkonzentrate zugesetzt werden.

### Synthesebeispiele:

### Beispiel 1

### Herstellung von Nitrilen der Formel IV

### 2-(4,6-Dimethoxypyrimidin-2-yl)oxy-3,3-dimethylbuttersäurenitril

29,5 g (0,14 mol) 2-Methylsulfonyl-4,6-dimethoxypyrimidin wurden in 180 ml absolutem Dimethylformamid suspendiert und 20 g (0,18 mol) 3,3-Dimethyl-2-hydroxybuttersäurenitril zugegeben. Bei 0°C wurden nun portionsweise 5,2 g Natriumhydrid (80%ig) zugegeben und die Mischung 3 Stunden bei 60°C gerührt. Nach dem Abkühlen wurden 10 g Essigsäure und 20 ml Wasser zugegeben und das Reaktionsgemisch auf 500 ml Wasser gegossen. Der entstandene Niederschlag wurde abgesaugt und getrocknet. Man erhielt 32,4 g eines weißen Pulvers mit einem Schmelzpunkt von 88-89°C (Ausbeute: 92 % d. Th.).

Analog lassen sich alle Verbindungen der Formel IV, bei denen Y Sauerstoff ist, aus Cyanhydrinen der Formel II und Verbindungen der Formel III herstellen.

### Beispiel 2:

### Herstellung von Thioamiden V

### 2-(4,6-Dimethoxypyrimidin-2-yl)oxy-3,3-dimethylthiobuttersäureamid

16,5 g (0,066 mol) 2-(4,6-Dimethoxypyrimidin-2-yl)oxy-3,3-dimethylbuttersäurenitril wurden in 150 ml absolutem Dimethylformamid gelöst und mit 2,5 g (0,033 mol) Diethylamin versetzt. Dann wurde bei 55°C ca. 3 Stunden Schwefelwasserstoff durch die Lösung geleitet und 1 Stunde bei dieser Temperatur nachgerührt. Das Reaktionsgemisch wurde auf 1,2 l Eiswasser gegossen und der gebildete Niederschlag abgesaugt und getrocknet. Man erhielt 18 g eines weißen Pulvers mit einem Schmelzpunkt von 187-189°C. (Ausbeute: 96 % d. Th.).

Analog lassen sich alle Nitrile der allgemeinen Formel IV in die Thioamide V umwandeln.

### Beispiel 3:

### Umwandlung der Thioamide in Thiocarbonsäurederivate I

### 2-(4,6-Dimethoxypyrimidin-2-yl) oxy-3,3-dimethylthiobuttersäure-thiomethylester

18 g (0,063 mol) 2-(4,6-Dimethoxypyrimidin-2-yl) oxy-3,3-dimethylthiobuttersäureamid wurden unter Argon in 200 ml absolutem Dichlormethan gelöst und bei 0°C 10 g (0,070 mol) Trimethyloxoniumtetrafluoroborat portionsweise zugegeben. Nach Rühren über Nacht bei Raumtemperatur wurde das Lösungsmittel abdestilliert, der Rückstand in 250 ml absolutem Methyl-t-butylether suspendiert und nach Zusatz von 70 ml absolutem Pyridin bei 0°C ca. 5 Stunden lang Schwefelwasserstoff durch die Lösung geleitet. Nach Rühren über Nacht bei Raumtemperatur wurde mit 100 ml Wasser versetzt und zweimal mit Essigester extrahiert. Die organische Phase wurde über Magnesiumsulfat getrocknet und anschließend das Lösungsmittel im Vakuum entfernt. Nach Umkristallisieren aus Ethanol erhielt man 6 g gelbe Kristalle mit einem Schmelzpunkt von 91 - 93°C. (Ausbeute 30 % d. Theorie).

Analog lassen sich alle Verbindungen der allgemeinen Formel I, bei denen Y Sauerstoff ist, darstellen.

### Beispiel 4:

Allgemeine Vorschrift zur Umsetzung von Carbonsäurederivaten der Formel VII mit Lawesson's Reagenz.

0,01 mol eines Carbonsäurederivats VII und 0,012 mol Lawesson's Reagenz werden in 10 ml Xylol ca. 8 Stunden auf 140°C erhitzt. Nach dem Abkühlen wird die Mischung direkt säulenchromatographisch gereinigt.

Entsprechend den Beispielen 1 bis 3 wurden die in Tabelle 1 aufgeführten Thiocarbonsäurederivate hergestellt.

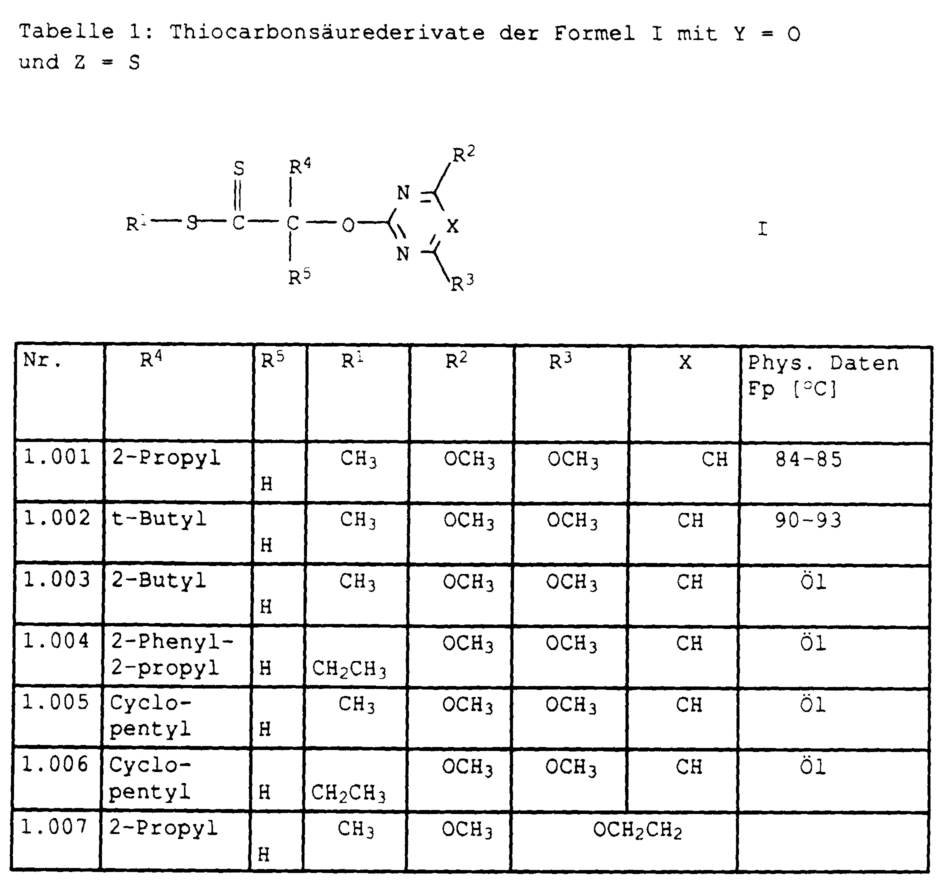

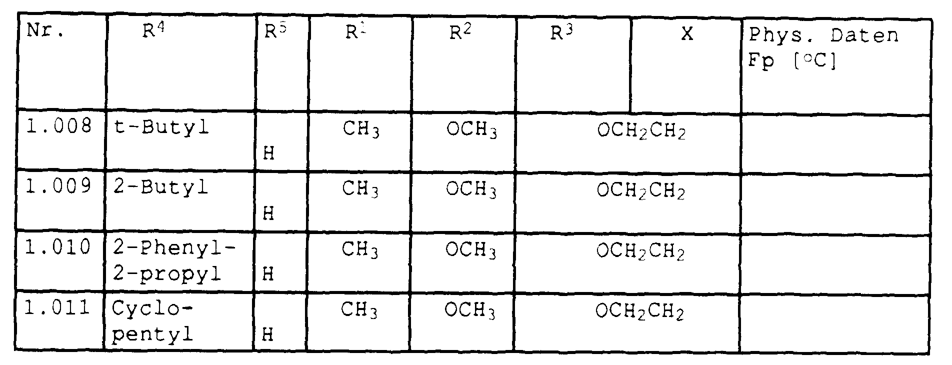

Die als Vorprodukte verwendeten Nitrile der Formel IV weisen die in Tabelle 2 angegebenen physikalischen Daten auf:

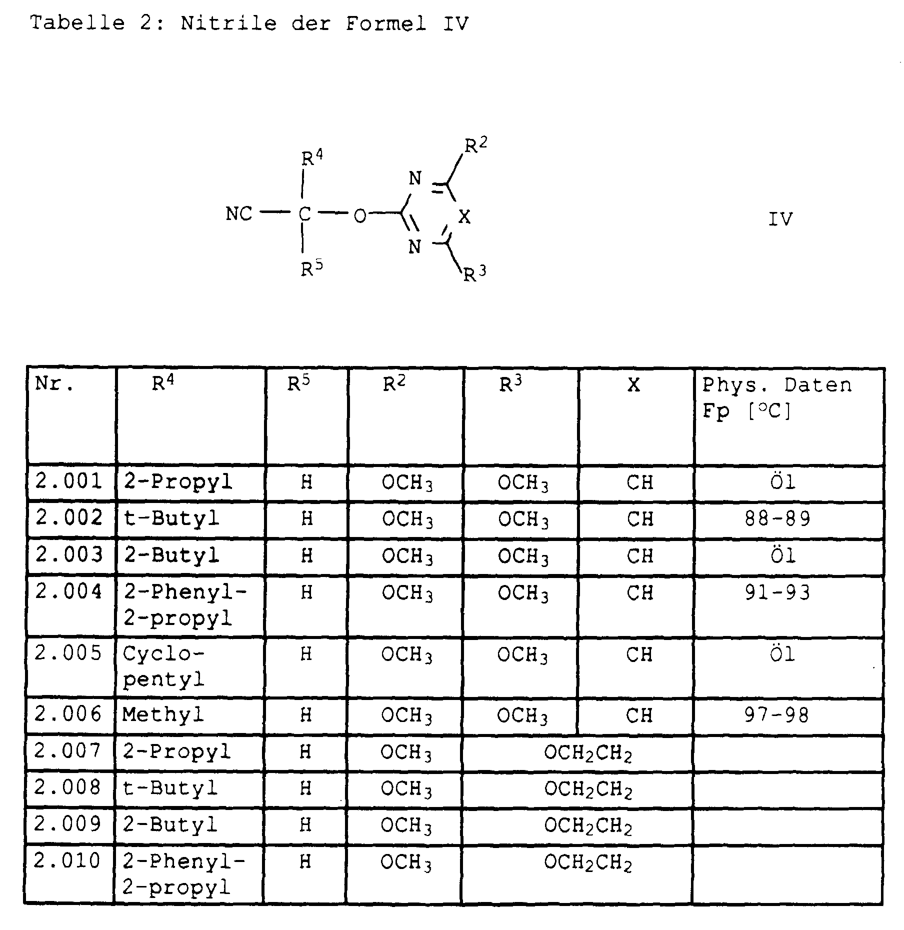

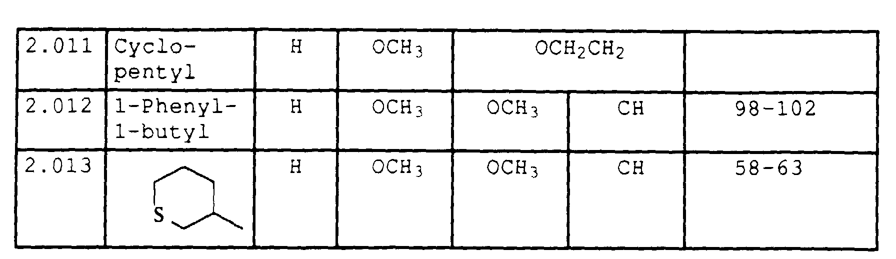

### Anwendungsbeispiele:

Die herbizide Wirkung der Verbindungen I ließ sich durch Gewächshausversuche zeigen:

Als Kulturgefäße dienten Plastikblumentöpfe mit lehmigem Sand mit etwa 3 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | |
|---|---|
| Lateinischer Name | Deutscher Name |
| Amaranthus retroflexus | Zurückgekrümmter Fuchsschwanz |
| Bromus Spp. | Trespe |
| Galium aparine | Klettenlabkraut |

Mit 3 kg/ha a.S. im Nachlaufverfahren eingesetzt, lassen sich mit dem Beispiel 1.003 breitblättrige unerwünschte Pflanzen und Schadgräser sehr gut bekämpfen.

Die Verbindung 1.005 zeigt bei einer Aufwandmenge von 0,5 kg/ha a.S. im Nachauflaufverfahren sehr gute Wirkung gegen die unerwünschten Pflanzen Chenopodium album, Galium aparine und Polygonum persicaria, wobei eine hohe Selektivität gegenüber den Kulturpflanzen Baumwolle und Reis zu beobachten ist.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen (ca. 8 cm Durchmesser; Volumen ca. 300 ml) angezogen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente 2-Chlorethyltrimethylammoniumchlorid (CCC).

Die Einzeldaten sind der folgenden Tabelle 3 zu entnehmen, wobei die Konzentration in kg Wirkstoff pro Hektar (kg/ha a.S.) angegeben ist.

**Tabelle 3:**

| Bioregulatorische Wirkung von Thiocarbonsäurederivaten I bei Nachauflaufanwendung | | | |
|---|---|---|---|
| Beispiel Nr. | Aufwandmenge kg/ha a.S. | relative Wuchshöhe | |
| | | Sommerweizen a) | Sommergerste b) |
| unbehandelt | - | 100 | 100 |
| CCC | 3 | 85 | 103 |
| 1.001 | 0,06 | 67 | 73 |
| 1.003 | 0,025 | 70 | 73 |
| 1.004 | 0,025 | 67 | 73 |
| 1.002 | 0,05 | 74 | 88 |

| | | | |
|---|---|---|---|
| a) Sorte "Star" | | | |
| b) Sorte "Alexis" | | | |

## Patentansprüche (Patentansprüche für folgende(n) Vertragsstaat(en): BE, CH, DE, ES, FR, GB, IT, LI, NL, SE)

1. Thiocarbonsäurederivate der allgemeinen Formel I, 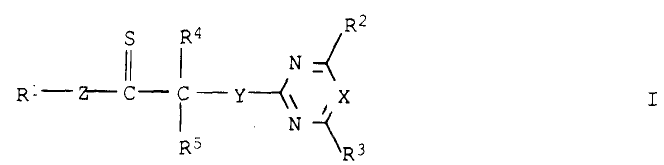 in der die Substituenten folgende Bedeutung haben:
R¹ eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, C₃-C₁₂-Cycloalkyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder ein bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
eine C₂-C₆-Alkylgruppe, welche in der 2-Position einen der folgenden Reste trägt: C₁-C₆-Alkoxyimino, C₃-C₆-Alkenyloxyimino, C₃-C₆-Halogenalkenyloxyimino oder Benzyloxyimino
eine C₃-C₁₂-Cycloalkylgruppe oder C₃-C₁₂-Cycloalkenylgruppe, welche ein bis fünf Halogenatome und/oder einen der folgenden Reste tragen kann:
C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
unsubstituiertes oder ein- bis dreifach durch C₁-C₄-Alkyl oder C₁-C₄-Alkoxy substituiertes oder ein- bis fünffach durch Halogen substituiertes Phenyl bedeutet;
R² Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR⁶, wobei R⁶ Wasserstoff bedeutet oder zusammen mit R³ eine 3- bis 4-gliedrige Alkylen- oder Alkenylenkette bildet, in der jeweils eine Methylengruppe durch Sauerstoff ersetzt ist und die Alkylen- bzw. Alkenylenkette durch C₁-C₄-Alkyl, Phenyl, C₁-C₄-Alkoxy oder C₁-C₄-Alkoxycarbonyl substituiert sein kann;
R³ Halogen, C₁-C₄-Alkyl, C₁-C₄-Halogenalkyl, C₁-C₄-Alkoxy, C₁-C₄-Halogenalkoxy, C₁-C₄-Alkylthio oder R³ ist mit R⁶ wie oben angegeben zu einem 5- oder 6-gliedrigen Ring verknüpft;
R⁴ eine C₁-C₁₀-Alkylgruppe, welche ein bis fünf Halogenatome und/oder einen der folgenden Reste tragen kann: C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₁-C₁₀-Alkylgruppe, welche ein bis fünf Halogenatome tragen kann und einen der folgenden Reste trägt: ein fünfgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio und/oder Phenyl;
eine C₃-C₁₂-Cycloalkyl- oder C₃-C₁₂-Cycloalkenylgruppe, die ein Sauerstoff- oder Schwefelatom enthalten kann und ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
eine C₃-C₆-Alkenyl- oder eine C₃-C₆-Alkinylgruppe, welche jeweils ein bis fünf Halogenatome und/oder einen der folgenden Reste tragen kann: C₁-C₄-Alkyl, C₁-C₄-Alkoxy, C₁-C₄-Alkylthio, Cyano, C₁-C₈-Alkylcarbonyl, C₁-C₈-Alkoxycarbonyl, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Phenyl oder Naphthyl, welches ein bis fünf Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
R⁴ und R⁵ bilden zusammen mit dem benachbarten Kohlenstoffatom einen 3- bis 6-gliedrigen Ring, der ein Sauerstoff- oder Schwefelatom enthalten kann und einen bis drei der folgenden Reste tragen kann: C₁-C₄-Alkyl, C₁-C₄-Halogenalkyl, C₁-C₄-Alkoxy, C₁-C₄-Halogenalkoxy und/oder C₁-C₄-Alkylthio;
R⁵ Wasserstoff, C₁-C₄-Alkyl, C₁-C₄-Halogenalkyl, C₁-C₄-Alkoxy, C₁-C₄-Halogenalkoxy, C₁-C₄-Alkylthio oder R⁵ ist mit R⁴ wie oben angegeben zu einem 3- bis 6-gliedrigen Ring verknüpft;
Y,Z Sauerstoff oder Schwefel.

2. Thiocarbonsäurederivate der Formel I gemäß Anspruch 1, in der R¹ Methyl oder Ethyl, R² Methoxy, R⁵ Wasserstoff, Y Sauerstoff bedeuten und R³, R⁴, X und Z die in Anspruch 1 genannte Bedeutung haben.

3. Herbizides Mittel, enthaltend ein Thiocarbonsäurederivat der Formel I gemäß den Ansprüchen 1 oder 2 und übliche inerte Zusatzstoffe.

4. Verfahren zur Bekämpfung unerwünschten Pflanzenwuches, dadurch gekennzeichnet, daß man die unerwünschten Pflanzen und/oder ihren Lebensraum mit einer herbizid wirksamen Menge eines Thiocarbonsäurederivats der Formel I gemäß Anspruch 1 behandelt.

5. Mittel zur Beeinflussung des Pflanzenwachstums, enthaltend ein Thiocarbonsäurederivat der Formel I gemäß den Ansprüchen 1 oder 2 und übliche inerte Zusatzstoffe.

6. Verfahren zur Regulierung des Pflanzenwachstums, dadurch gekennzeichnet, daß man eine bioregulatorisch wirksame Menge eines Thiocarbonsäurederivats der Formel I gemäß Anspruch 1 auf die Samen, die Pflanzen und/oder ihren Lebensraum einwirken läßt.

7. Nitrile der allgemeinen Formel IV, 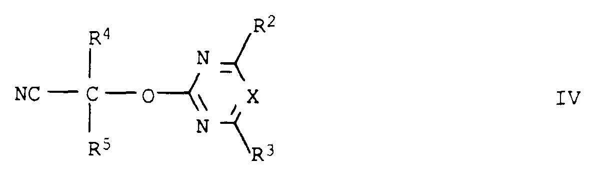 in der R², R³, R⁴, R⁵ und X die in Anspruch 1 genannte Bedeutung haben.

8. Verfahren zur Herstellung von Thiocarbonsäurederivaten der Formel I, in der Y für Sauerstoff steht, dadurch gekennzeichnet, daß man Nitrile der Formel IV, 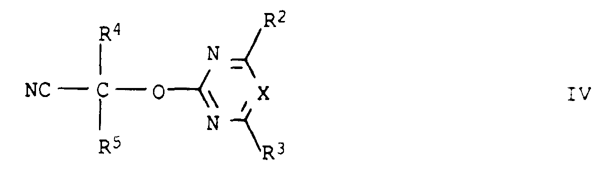 in der die Reste R² bis R⁵ und X die in Anspruch 1 genannte Bedeutung haben, mit Schwefelwasserstoff zu den Thioamiden V 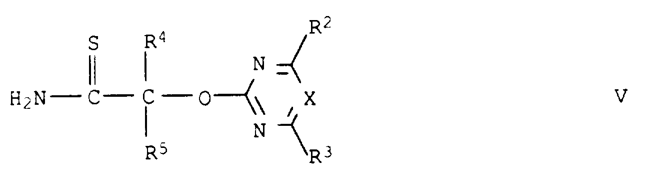 umsetzt und die Thioamide V mit Verbindungen der Formel VI,
R¹-A VI
in der R¹ die in Anspruch 1 genannte Bedeutung hat und A für eine nukleofuge Abgangsgruppe steht, zur Reaktion bringt und anschließend mit Schwefelwasserstoff in die Verbindungen I überführt.

9. Verfahren zur Herstellung von Thiocarbonsäurederivaten der Formel I gemäß Anspruch 1 durch Umsetzung entsprechender Carbonsäurederivate der Formel VII 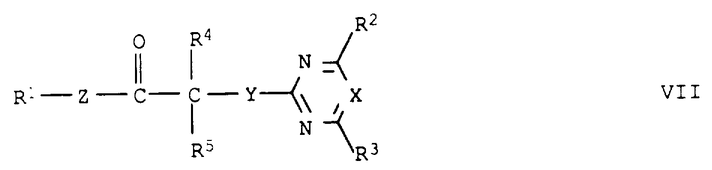 mit Lawesson's Reagenz in allgemein bekannter Weise.

## Claims (Claims for the following Contracting State(s): BE, CH, DE, ES, FR, GB, IT, LI, NL, SE)

1. A thiocarboxylic acid derivative of the formula I 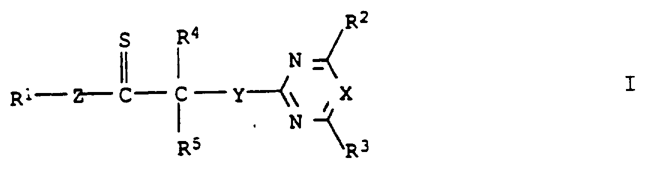 where
R¹ is C₁-C₁₀-alkyl which may carry from one to five halogen atoms and/or one of the following radicals: C₁-C₄-alkoxy, C₁-C₄-alkylthio, cyano, C₁-C₈-alkylcarbonyl, C₁-C₈-alkoxycarbonyl, C₃-C₁₂-cycloalkyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₁-C₁₀-alkyl which may carry from one to five halogen atoms and carries one of the following radicals: a five-membered heteroaromatic structure containing from one to three nitrogen atoms and/or one sulfur or oxygen atom, which may carry from one to four halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
C₂-C₆-alkyl which carries one of the following radicals in the 2-position: C₁-C₆-alkoximino, C₃-C₆-alkenyloximino, C₃-C₆-haloalkenyloximino or benzyloximino;
C₃-C₁₂-cycloalkyl or C₃-C₁₂-cycloalkenyl which may carry from one to five halogen atoms and/or one of the following radicals: C₁-C₄-alkyl, C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₃-C₆-alkenyl or C₃-C₆-alkynyl which may carry from one to five halogen atoms and/or one of the following radicals: C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
phenyl which is unsubstituted or monosubstituted to trisubstituted by C₁-C₄-alkyl or C₁-C₄-alkoxy or monosubstituted to pentasubstituted by halogen;
R² is halogen, C₁-C₄-alkyl, C₁-C₄-haloalkyl, C₁-C₄-alkoxy, C₁-C₄-haloalkoxy or C₁-C₄-alkylthio;
X is nitrogen or CR⁶, where R⁶ is hydrogen or, together with R³, forms a 3-membered or 4-membered alkylene or alkenylene chain, in each of which a methylene group is replaced with oxygen and each of which may be substituted by C₁-C₄-alkyl, phenyl, C₁-C₄-alkoxy or C₁-C₄-alkoxycarbonyl;
R³ is halogen, C₁-C₄-alkyl, C₁-C₄-haloalkyl, C₁-C₄-alkoxy, C₁-C₄-haloalkoxy or C₁-C₄-alkylthio or R³ is bonded to R⁶ as stated above to form a 5-membered or 6-membered ring; R⁴ is C₁-C₁₀-alkyl which may carry from one to five halogen atoms and/or one of the following radicals:
C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₁-C₁₀-alkyl which may carry from one to five halogen atoms and carries one of the following radicals: a five-membered heteroaromatic structure containing from one to three nitrogen atoms and/or one sulfur or oxygen atom, which may carry from one to four halogen atoms and/or one or two of the following radicals: C₁-C₄-alkyl, C₁-C₄-haloalkyl, C₁-C₄-alkoxy, C₁-C₄-haloalkoxy, C₁-C₄-alkylthio and/or phenyl;
C₃-C₁₂-cycloalkyl or C₃-C₁₂-cycloalkenyl, each of which may contain an oxygen or sulfur atom and may carry from one to five halogen atoms and/or one of the following radicals: C₁-C₄-alkyl, C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
C₃-C₆-alkenyl or C₃-C₆-alkynyl, each of which may carry from one to five halogen atoms and/or one of the following radicals: C₁-C₄-alkyl, C₁-C₄-alkoxy, C₁-C₄-alkylthio, cyano, C₁-C₈-alkylcarbonyl, C₁-C₈-alkoxycarbonyl, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
a five-membered or six-membered heteroaromatic structure containing from one to three nitrogen atoms and/or one sulfur or oxygen atom, which may carry from one to four halogen atoms and/or one or two of the following radicals: C₁-C₄-alkyl, C₁-C₄-haloalkyl, C₁-C₄-alkoxy, C₁-C₄-haloalkoxy, C₁-C₄-alkylthio, phenyl, phenoxy or phenylcarbonyl, where the phenyl radicals in turn may carry from one to five halogen atoms and/or from one to three of the following radicals: C₁-C₄-alkyl, C₁-C₄-haloalkyl, C₁-C₄-alkoxy, C₁-C₄-haloalkoxy and/or C₁-C₄-alkylthio;
phenyl or naphthyl which may carry from one to five halogen atoms and/or one or two of the following radicals: C₁-C₄-alkyl, C₁-C₄-haloalkyl, C₁-C₄-alkoxy, C₁-C₄-haloalkoxy and/or C₁-C₄-alkylthio;
R⁴ and R⁵ together with the adjacent carbon atom form a 3-membered to 6-membered ring which may contain an oxygen or sulfur atom and may carry from one to three of the following radicals: C₁-C₄-alkyl, C₁-C₄-haloalkyl, C₁-C₄-alkoxy, C₁-C₄-haloalkoxy and/or C₁-C₄-alkylthio;
R⁵ is hydrogen, C₁-C₄-alkyl, C₁-C₄-haloalkyl, C₁-C₄-alkoxy, C₁-C₄-haloalkoxy or C₁-C₄-alkylthio, or R⁵ is bonded to R⁴ as stated above to form a 3-membered to 6-membered ring,
and
Y and Z are each oxygen or sulfur.

2. A thiocarboxylic acid derivative of the formula I as claimed in claim 1, wherein R¹ is methyl or ethyl, R² is methoxy, R⁵ is hydrogen, Y is oxygen and R³, R⁴, X and Z have the meanings stated in claim 1.

3. A herbicide containing a thiocarboxylic acid derivative of the formula I as claimed in claim 1 or 2 and conventional inert additives.

4. A method for controlling undesirable plant growth, wherein the undesirable plants and/or their habitat is or are treated with a herbicidal amount of a thiocarboxylic acid derivative of the formula I as claimed in claim 1.

5. A plant growth regulator containing a thiocarboxylic acid derivative of the formula I as claimed in claim 1 or 2 and conventional inert additives.

6. A method for regulating plant growth, wherein a bioregulatory amount of a thiocarboxylic acid derivative of the formula I as claimed in claim 1 is allowed to act on the seeds, the plants and/or their habitat.

7. A nitrile of the general formula IV 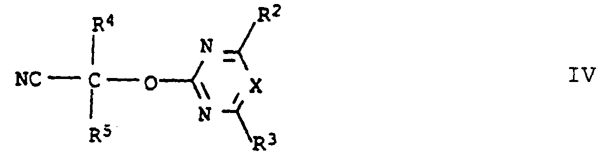 where R², R³, R⁴, R⁵ and X have the meanings stated in claim 1.

8. A process for the preparation of a thiocarboxylic acid derivative of the formula I where Y is oxygen, wherein a nitrile of the formula IV 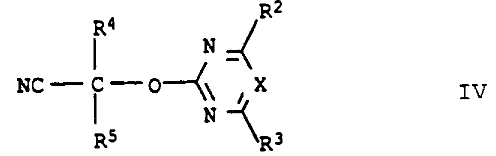 where R² to R⁵ and X have the meanings stated in claim 1, is reacted with hydrogen sulfide to give a thioamide V 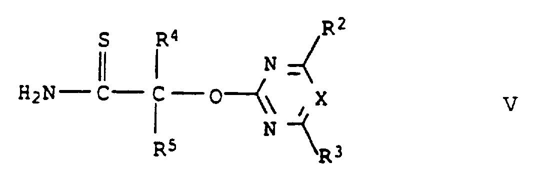 the thioamide V is reacted with a compound of the formula VI
R¹-A VI
where R¹ has the meanings stated in claim 1 and A is a nucleofugic leaving group, and the product is then converted with hydrogen sulfide into a compound I.

9. A process for the preparation of a thiocarboxylic acid derivative of the formula I as claimed in claim 1 by reacting a corresponding carboxylic acid derivative of the formula VII 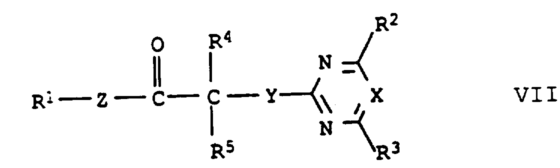 with Lawesson's reagent in a generally known manner.

## Revendications (Revendications pour l'(les) Etat(s) contractant(s) suivant(s): BE, CH, DE, ES, FR, GB, IT, LI, NL, SE)

1. Dérivés d'acides thiocarboxyliques répondant à la formule générale I 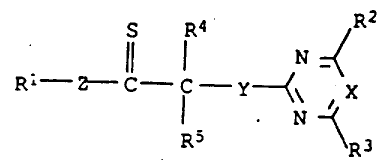 dans laquelle les symboles ont les significations suivantes :
R¹ : un groupe alkyle en C1-10 qui peut porter à un cinq atomes d'halogènes et/ou un des substituants suivants : alcoxy en C1-C4, alkythio en C1-C4, cyano, (alkyle en C1-C8)- carbonyle, (alcoxy en C1-C8) carbonyle en C3-C12, phényle, phénoxy ou phénylcarbonyle, les groupes phényle pouvant eux-mêmes porter à cinq atomes d'halogènes et/ou un à trois des substituants suivants : alkyle en C1-C4, halogénoalkyle en C1-C4, alcoxy en C1-C4, halogénoalcoxy en C1-C4 et/ou alkylthio en C1-C4 ;
un groupe alkyle en C1-C10 qui peut porter un à cinq atomes d'halogènes et porte un des substituants suivants : un groupe hétéroaromatique à cinq chaînons contenant un à trois atomes d'azote et/ou un atome de soufre ou d'oxygène, qui peut porter un à quatre atomes d'halogènes ou un à deux de substituants suivants :
alkyle en C1-C4, halogénoalkyle en C1-C4, alcoxy en C1-C4, halogénoalcoxy en C1-C4, halogénoalcoxy en C1-C4 et/ou alkylthio en C1-C4 ;
un groupe alkyle en C2-C6 qui porte en position 2 l'un des substituants suivants : alcoxyimino en C1-C6, alcényloxyimino en C3-C6, halogénoalcényloxyimino en C3-C6 ou benzyloxymino,
un groupe cycloalkyle en C3-C12 ou cycloalcényle en C3-C12 qui peut porter un à cinq atomes d'halogènes et/ou des substituants suivants : alkyle en C1-C4, alcoxy en C1-C4, alkylthio en C1-C4, cyano, (alkyle en C1-C8)- carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants :
alkyle en C1-C4, halogénoalkyle en C1-C4, alcoxy en C1-C4, halogénoalcoxy en C1-C4 et/ou alkylthio en C1-C4 ;
un groupe alcényle en C3-C6 ou alcynyle en C3-C6 qui peut porter un à cinq atomes d'halogènes et/ou un des substituants suivants : alcoxy en C1-C4, alkylthio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4,alcoxy en C1-C4,halogénoalcoxy en C1-C4 et/ou alkylthio en C1-C4 ;
un groupe phényle non substitué ou portant un à trois substituants alkyle en C1-C4 ou alcoxy en C1-C4 ou un à cinq substituants halogéno;
R² : un halogène, un groupe alkyle en C1-C4, halogénoalkyle en C1-C4, alcoxy en C1-C4, halogénoalcoxy en C1-C4 ou alkythio en C1-C4 ;
X : l'azote ou CR⁶, R⁶ représentant l'hydrogène ou formant avec R³ une chaîne alkylène ou alcénylène à trois ou quatre chaînons dans laquelle un groupe méthylène peut être remplacé par l'oxygène, la chaîne alkylène ou alcénylène pouvant porter des substituants alkyle en C1-C4, phényle, alcoxy en C1-C4 ou (alcoxy en C1-C4 carbonyle ;
R³ : un halogène, un groupe alkyle en C1-C4, halogénoalkyle en C1-C4, alcoxy en C1-C4, halogénoalcoxy en C1-C4, alkylthio en C1-C4, ou bien R³ est relié avec R⁶ comme indiqué ci-dessus avec formation d'un cycle à cinq ou six chaînons ;
R⁴ : un groupe alkyle en C1-C10 qui peut porter un à cinq atomes d'halogènes et/ou un des substituants suivants : alcoxy en C1-C4, alkylthio en C1-C4, cyano, (alkyle en Cl-C8)carbonyle, (alcoxy en C1-C8)carbonyle, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkythio en C1-C4 ;
un groupe alkyle en C1-C10 qui peut porter un à cinq atomes d'halogènes et porte un des substituants suivants : un groupe hétéroaromatique à cinq chaînons contenant un à trois atomes d'azote et/ou un atome de soufre ou d'oxygène, qui peut porter un à quatre atomes d'halogènes et/ou un ou deux des substituants suivants : alkyle en C1-C4, halogénoalkyle en C1-C4, alcoxy en C1-C4, halogénoalcoxy en C1-C4, alkythio en C1-C4 et/ou phényle ;
un groupe cycloalkyle en C3-C12 ou cycloalcényle en C3-C12 qui peut contenir un atome d'oxygène ou de soufre et qui peut porter un à cinq atomes d'halogènes et/ou un des substituants suivants : alkyle en C1-C4, alcoxy en C1-C4, alkythio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter à cinq atomes d'halogènes et/ou un à trois substituants suivants : alkyle en C1-C4, halogénoalkyle en C1-C4, alcoxy en C1-C4, halogénoalcoxy en C1-C4 et/ou alkythio en C1-C4 ;
un groupe alcényle en C3-C6 ou alcynyle en C3-C6, qui peut porter un à cinq atomes d'halogènes et/ou un des substituants suivants :
alkyle en C1-C4, alcoxy en C1-C4, alkythio en C1-C4, cyano, (alkyle en C1-C8)carbonyle, (alcoxy en C1-C8)carbonyle, phényle, phénoxy ou phénylcarbonyle, les groupes phényle pouvant eux-mêmes porter un à cinq atomes d'halogènes et/ou à trois des substituants suivants :
alkyle en C1-C4, halogénoalkyle en C1-C4, alcoxy en C1-C4, halogénoalcoxy en C1-C4 et/ou alkylthio en C1-C4 ;
un groupe hétéroaromatique à cinq ou six chaînons contenant un à trois atomes d'azote et/ou un atome de soufre ou d'oxygène, qui peut porter un à quatre atomes d'halogènes et/ou un ou deux substituants suivants :
alkyle en C1-C4, halogénoalkyle en C1-C4, alcoxy en C1-C4, halogénoalcoxy en C1-C4, alkylthio en C1-C4, phényle, phénoxy ou phénylcarbonyle, les groupes phényle pouvant eux-mêmes porter un à cinq atomes d'halogènes et/ou un à trois des substituants suivants : alkyle en C1-C4, halogénoalkyle en C1-C4, alcoxy en C1-C4, halogénoalcoxy en C1-C4 et/ou alkythio en C1-C4 ;
un groupe phényle ou naphtyle qui peut porter un à cinq atomes d'halogènes et/ou un ou deux substituants suivants : alkyle en C1-C4, halogénoalkyle en C1-C4, alcoxy en C1-C4, halogénoalcoxy en C1-C4 et/ou alkylthio en C1-C4 ;
R⁴ et R⁵ forment ensemble et avec l'atome de carbone voisin un cycle de trois à six chaînons qui peut contenir un atome d'oxygène ou de soufre et peut porter un à trois des substituants suivants : alkyle en C1-C4, halogénoalkyle en C1-C4, alcoxy en C1-C4, halogénoalcoxy en C1-C4 et/ou alkylthio en C1-C4 ;
R⁵ : l'hydrogène, un groupe alkyle en C1-C4, halogénoalkyle en C1-C4, alcoxy en C1-C4, halogénoalcoxy en C1-C4, alkylthio en C1-C4 ou bien R⁵ est relié avec R⁴ comme indiqué ci-dessus avec formation d'un cycle de trois à six chaînons ;
Y, Z : l'oxygène ou le soufre.

2. Dérivés d'acides thiocarboxyliques de formule I selon la revendication 1, pour lesquels R¹ représente un méthyle ou éthyle, R² un groupe méthoxy, R⁵ l'hydrogène, Y l'oxygène, et R³, R⁴, X, Z ont les significations indiquées dans la revendication 1.

3. Produit herbicide contenant un dérivé d'acide thiocarboxylique de formule I selon les revendications 1 et 2 avec des additifs inertes usuels.

4. Procédé pour combattre les croissances de végétaux indésirables, caractérisé par le fait que l'on traite les végétaux indésirables et/ou leur habitat par une quantité herbicide efficace d'un dérivé d'acide thiocarboxylique de formule I selon revendication 1.

5. Produit pour agir sur la croissance des végétaux, contenant un dérivé d'acide thiocarboxylique de formule I selon les revendications 1 ou 2 avec des additifs inertes usuels.

6. Procédé pour la régulation de la croissance des végétaux, caractérisé par le fait que l'on fait agir une quantité biorégulatrice efficace d'un dérivé d'acide thiocarboxylique de formule I selon revendication 1 sur les semences, les végétaux et/ou leur habitat.

7. Nitriles de formule générale IV 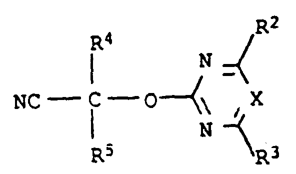 dans laquelle R², R³, R⁴, R⁵ et X ont les significations indiquées dans la revendication 1.

8. Procédé de préparation des dérivés d'acides thiocarboxyliques de formule I dans laquelle Y représente l'oxygène, caractérisé par le fait que l'on fait réagir des nitriles de formule IV. 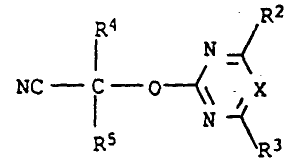 dans laquelle les symboles R² à R⁵ et X ont les significations indiquées dans la revendication 1, avec le sulfure d'hydrogène, ce qui donne des thioamides V 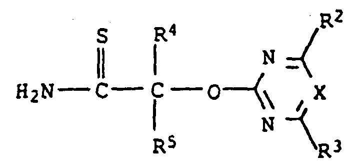 qu'on fait réagir avec des composés de formule VI
R¹A
dans laquelle R¹ a les significations indiquées dans la revendication 1 et A représente un groupe éliminable nucléofuge, après quoi on convertit, à l'aide du sulfure d'hydrogène, en les composés I.

9. Procédé de préparation des dérivés d'acides thiocarboxyliques de formule I selon revendication 1, par réaction, de manière connue en soi, entre les dérivés d'acides carboxyliques correspondants répondant à la formule VII 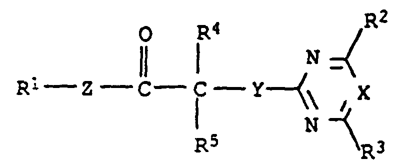 avec le réactif de Lawesson
